# EUROPEAN PATENT SPECIFICATION

(11) **EP 3 829 620 B1**
(45) Date of publication and mention of the grant of the patent: **29.05.2024**
(21) Application number: 19769570.3
(22) Date of filing: 31.07.2019
(51) Int. Cl.: A61K 38/17, C07K 14/47, A61P 21/06

(54) **NEW MYOKINES AND USES THEREOF**
NEUE MYOKINE UND VERWENDUNGEN DAVON
NOUVELLES MYOKINES ET LEURS UTILISATIONS

(30) Priority: 02.08.2018 EP 18187131
(43) Date of publication of application: 09.06.2021
(73) Proprietor: Novartis AG, 4056 Basel (CH)
(72) Inventor: HOEFLING, Holger, 4002 Basel (CH); KELLER, Hansjörg, 4002 Basel (CH); LEMIRE, Sophie, 4002 Basel (CH); SUMMERMATTER, Serge, 4002 Basel (CH)
(74) Representative: Badur, Ralf
(86) International application number: PCT/IB2019/056537
(87) International publication number: WO 2020/026167

(56) References cited:
- WO-A2-03/004532
- Silberberg, Amanda Beth; Waddell, Thomas K.: "Evaluating the Impact of Novel Bone Marrow Cell-secreted Factors, C19orf10 and C1orf54 , on Immunomodulation, Lung Cell Proliferation, and Resistance to Injury (Masters thesis)", 2017, Proquest LLC, Ann Arbor Michigan, XP002788439, ISBN: 978-1-369-68175-8 first paragraph of Section 5.2

## Description

### FIELD OF THE INVENTION

The present invention relates to chromosome 1 open reading frame 54 (C1orf54) polypeptides and variants thereof. The invention provides human chromosome 1 open reading frame 54 (C1orf54) polypeptides and variants thereof with myokine activity. The invention provides human chromosome 1 open reading frame 54 (C1orf54) polypeptides and variants thereof for use in therapy. In particular, the invention provides C1orf54 polypeptides and variants thereof with myokine activity for treating, ameliorating or preventing muscle-related disorders.

### BACKGROUND OF THE INVENTION

Many common acute and chronic conditions are associated with musculoskeletal impairments. For example, patients with disuse atrophy following surgery, injuries of the lower extremities, intensive care unit (ICU) myopathy, or aging-related health restrictions typically experience progressive loss of muscle mass and function. Reductions in muscle mass or strength, often in conjunction with impaired regeneration, have adverse consequences, such as severe functional limitations, an increased risk of falling, disability, and reduced quality of life. Musculoskeletal impairments are common in men and women of all ages across all socio-demographic strata of our modern society and affect hundreds of millions of people globally. The prevalence of musculoskeletal impairments increases markedly with age, and many are additionally affected by prevailing lifestyle factors such as a low physical activity. Therefore, these conditions represent large, unmet medical and socioeconomic needs. Current treatments are often restricted to the management of symptoms, rather than targeting prevention or cure.

Skeletal muscle is a tissue with high plasticity and high responsiveness to exercise. Satellite cells (muscle precursor cells) are typically activated upon exercise and injuries and play a key role in the remodeling of muscle tissue. Exercise is a highly efficacious therapy *inter alia* for psychiatric diseases, neurological diseases, metabolic diseases, cardiovascular diseases, pulmonary diseases, musculoskeletal disorders, and cancer (Pedersen and Saltin (2015) Scand J Med Sci Sports 25 Suppl 3, 1-72). It is believed that muscle-derived secreted factors are involved in the mediation of beneficial effects of exercise (Giudice and Taylor (2017) Curr Opin Pharmacol 34, 49-55). Such factors include a variety of cytokines and peptides, which can exert autocrine, paracrine or endocrine effects, and are collectively referred to as 'myokines' (Pedersen and Febbraio (2012) Nat Rev Endocrinol 8, 457-465). Analyses of the skeletal muscle secretome revealed hundreds of myokines that are secreted in response to exercise or contraction. However, not all myokines have a positive effect on muscle growth. The myokine myostatin, for instance, inhibits myogenesis and is a negative muscle regulator (Elkina et al. (2011) J Cachexia Sarcopenia Muscle 2, 143-151). Furthermore, for affected patients it is generally difficult to exert strenuous physical activities. Although known muscle anabolic agents increase muscle mass which can subsequently lead to increased muscle strength, anabolic agents can result in variety of undesired side effects: steroid use has been associated with high blood pressure (Urhausen A, Albers T, Kindermann W. Heart Br Card Soc. 2004;90(5):496-501), decreased function of the heart's ventricles (Pope HG, Kanayama G, Athey A, Ryan E, Hudson JI, Baggish A. Am J Addict Am Acad Psychiatr Alcohol Addict. 2014;23(4):371-377), cardiovascular diseases such as heart attacks (Vanberg P, Atar D. Handb Exp Pharmacol. 2010;(195):411-457), artery damage (Baggish AL, Weiner RB, Kanayama G, et al. 2017;135(21):1991-2002) and strokes (El Scheich T, Weber A-A, Klee D, Schweiger D, Mayatepek E, Karenfort M. J Pediatr Endocrinol Metab JPEM. 2013;26(1-2):161-165). Furthermore, increase in muscle volume does not necessarily translate into increased muscle strength (Hayashida I, Tanimoto Y, Takahashi Y, Kusabiraki T, Tamaki J. PLoS One. 2014 Nov 3;9(11):e111810). Hence, there is a strong medical need for the provision of myokine-based treatments that mimic the effects of exercise with a direct qualitative effect on muscle strength.

### SUMMARY OF THE INVENTION

The invention is set out in the appended set of claims

### BRIEF DESCRIPTION OF THE DRAWINGS

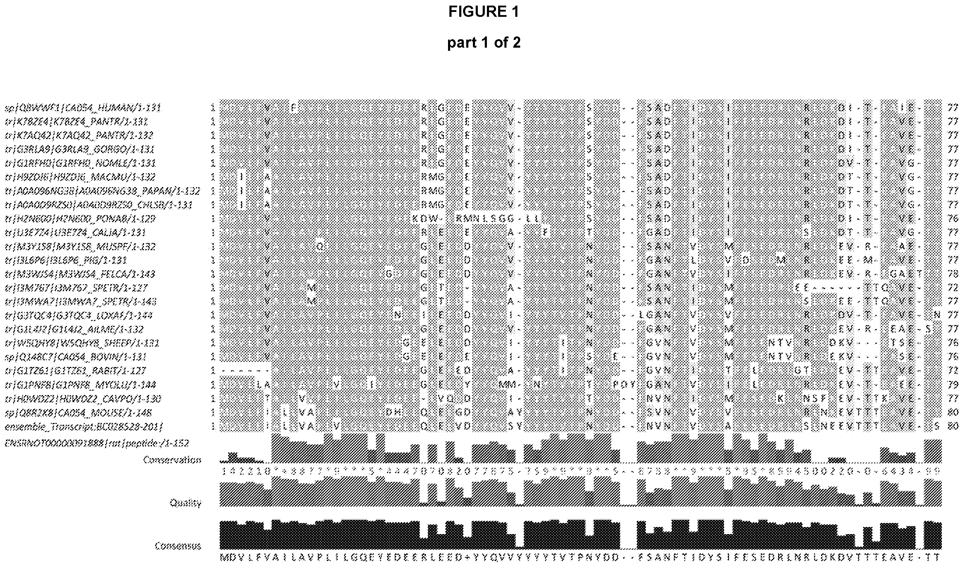

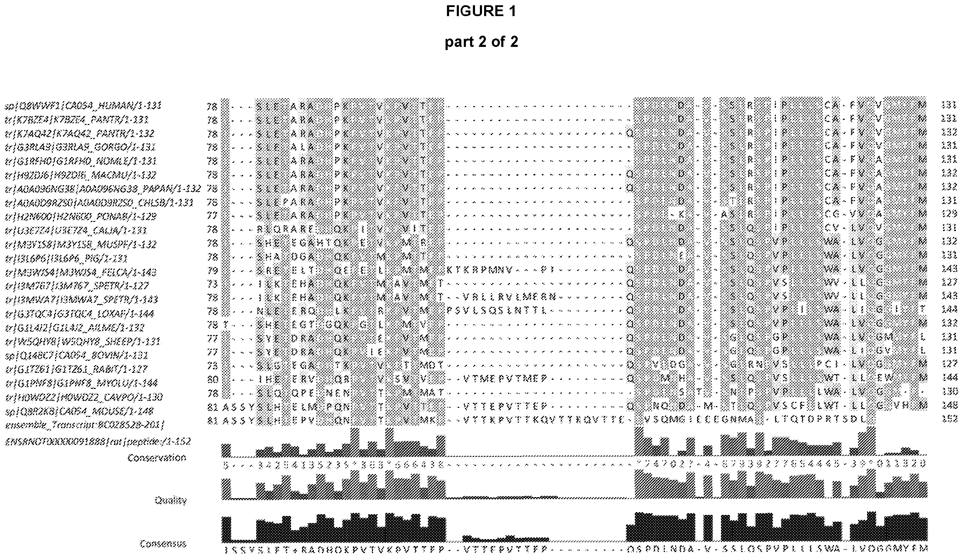

***Figure 1*****:** Alignment of human C1orf54 (UniProt ID Q8WWF1) (SEQ ID NO:1) with homologous proteins (SEQ ID NOs:46-68, respectively, in order of appearance). Sequences were retrieved from www.uniprot.org (The UniProt Consortium (2017), Nucleic Acids Research, 45(D1):D158-D169) or www.ensembl.org (Zerbino et al. (2018) Nucleic Acids Research, 46(D1):D754-D761), respectively, on August 18, 2015 and aligned in Jalview (Waterhouse et al. (2009) Bioinformatics, 25(9):1189-1191) using the MUSCLE algorithm (Edgar (2004) Nucleic Acids Research, 32(5):1792-1797).

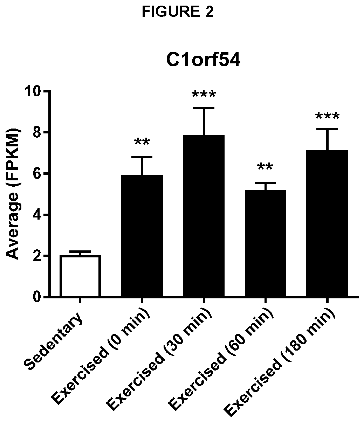

***Figure 2******:*** Increase in *C1orf54* mRNA levels in quadriceps of exercised compared to sedentary mice. *C1orf54* mRNA levels were elevated immediately after exercise and remained elevated at 30, 60 and 180 minutes after cessation of exercise. n= 7-8 mice/group. All values are expressed as mean ± SEM. Differential analysis was performed on counts per million reads using limma-voom Bioconductor package. All p-values were adjusted using Benjamini and Hochberg multiple testing correction. Statistically significant differences from the sedentary group are indicated as follows: **p < 0.01, ***p<0.001. FPKM = Fragments per kilobase of transcript per million mapped reads.

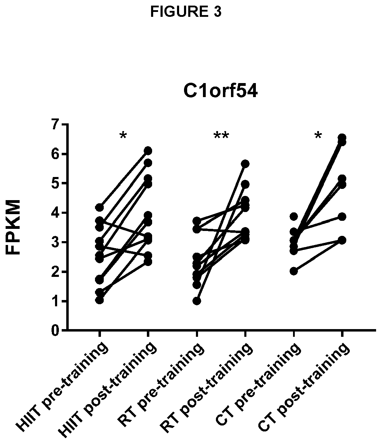

***Figure 3******:*** Increase in *C1ORF54* mRNA levels in human skeletal muscle from young subjects in response to different exercise paradigms alone or in combination. Individual values pre- and post-training are shown for each subject from the group of young participants. n=7-11 participants/group. One subject in the CT group was excluded from the statistical analysis as no post-training value was available. All p-values were adjusted using Benjamini and Hochberg multiple testing correction. Statistically significant differences from pre-training are indicated as follows: *p < 0.05, **p<0.01. FPKM = Fragments per kilobase of transcript per million mapped reads, HilT = high-intensity aerobic interval training, RT = resistance training, CT = combined exercise training.

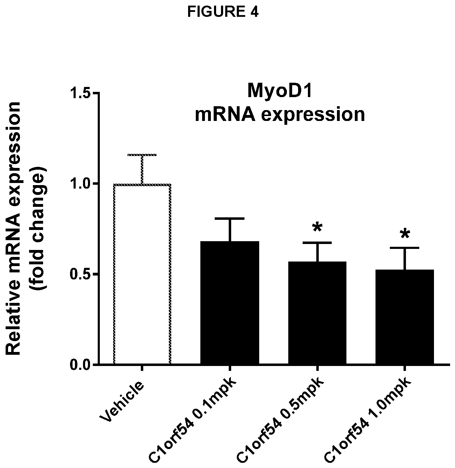

***Figure 4******:*** Changes in *Myod1* mRNA in gastrocnemius muscle of mice treated with vehicle (PBS) or isolated Protein comprising amino acid sequence SEQ ID NO:25 formulated in vehicle at the indicated dose. n= 5 mice/group. All values are expressed as mean ± SEM. Statistically significant differences are assessed by one-way ANOVA followed by Fisher's LSD and indicated as follows: *p < 0.05. Mpk = milligram per kilogram body weight.

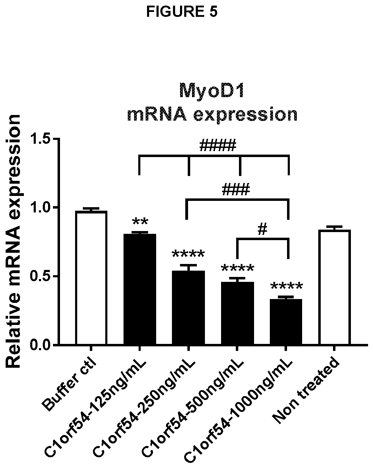

***Figure 5******:*** Dose-dependent decrease in *MYOD1* mRNA in human myoblasts upon treatment with recombinant full-length human C1orf54 (SEQ ID NO:2, My Biosources, ref. MBS1284901) with the indicated dose or vehicle for 6 hours. Values are expressed as mean ± SEM, n= 4 per group. Statistically significant differences are assessed by one-way ANOVA and Tukey's post hoc test and indicated as follows: **p < 0.01, ***p<0.001 compared to buffer control; # p<0.05, ## p < 0.01, ### p<0.001 compared as shown in the graph.

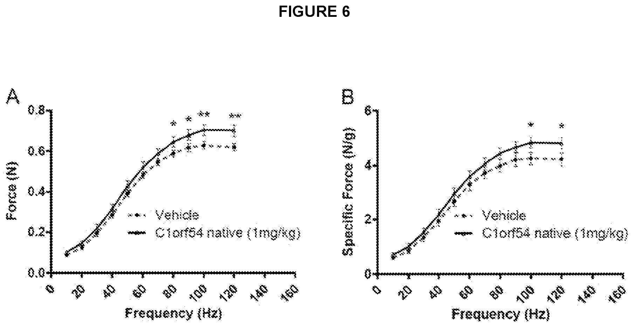

***Figure 6A******:*** Increase in absolute muscle force in response to treatment with vehicle or 1 mg/kg C1orf54 isolated protein comprising amino acid sequence SEQ ID NO:25.
***Figure6B******:*** Increase in specific force in response to treatment with vehicle or 1 mg/kg C1orf54 isolated protein comprising amino acid sequence SEQ ID NO:25. Specific muscle force is calculated by normalizing absolute muscle force for gastrocnemius weight. For panel A and B, n= 9 mice/group. All values are expressed as mean ± SEM. Statistically significant differences are assessed by multiple unpaired t-test comparison and indicated as follows: *p < 0.05 and **p < 0.01.

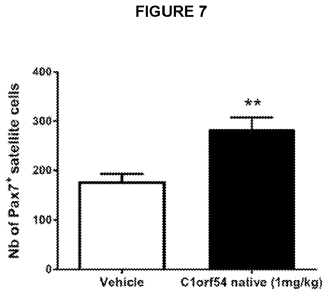

***Figure 7******:*** Number of Pax7 positive satellite cells in mouse gastrocnemius following treatment with vehicle or C1orf54 isolated protein comprising amino acid sequence SEQ ID NO:25. Values are expressed as mean ± SEM, n= 9-10 mice/group. Statistically significant differences are assessed by unpaired t-test and indicated as follows: **p < 0.01.

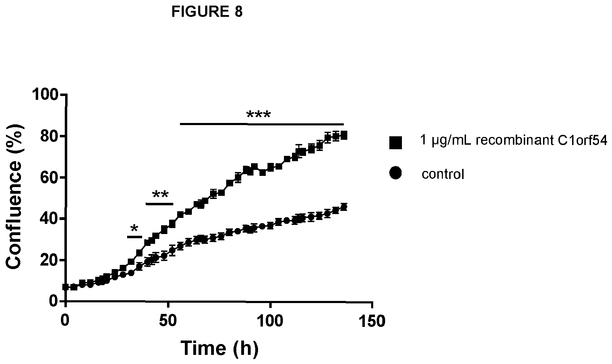

***Figure 8******:*** Growth curves (in percent confluence) of primary human myoblasts treated with 1 µg/mL recombinant full-length human C1orf54 protein (SEQ ID NO:2, My Biosources, ref. MBS1284901) (squares) and solvent control (circles) in growth medium containing 1% FBS. Values are expressed as mean ± SEM, n=6 per group. Statistically significant differences are assessed by multiple unpaired t-test comparison and indicated as follows: *p < 0.05, **p < 0.01 and ***p<0.001.

### GENERAL DEFINITIONS

In order that the present invention may be more readily understood, certain terms are first defined. Additional definitions are set forth throughout the detailed description.

The term "comprising" means "including" as well as "consisting" e.g. a composition "comprising" X may consist exclusively of X or may include something additional e.g. X + Y.

The present invention includes C1 orf54 polypeptides and variants thereof with biological activity. As used herein, the term "chromosome 1 open reading frame 54 (C1orf54) polypeptide" refers to polypeptides encoded by chromosome 1 open reading frame 54 (C1orf54) genes, particularly preferred is the human chromosome 1 open reading frame 54 (C1orf54) (UniGene ID:141426, UniGene Hs.91283, NM_024579.3, NP_078855.2, Uniprot accession no. Q8WWF1 with SEQ ID NO:1, which herein refers to C1orf54 polypeptide comprising a signal peptide), preferably having an amino acid sequence as set out in SEQ ID NO:2, which herein refers to C1orf54 polypeptide without signal peptide.

For the sake of clarity and consistency, the numbering of amino acid residues in C1 orf54 proteins throughout this application and in the claims is based on the wild-type C1orf54 protein sequence numbering of C1orf54 (Uniprot accession no. Q8WWF1) without signal peptide (i.e. SEQ ID NO:2). Herein, this SEQ ID NO:2 is referred to as the "full-length sequence".

The terms "C1orf54 polypeptide" also includes variants of the C1 orf54 polypeptides disclosed herein (e.g. C1orf54 polypeptides comprising amino acid SEQ ID NO:3 or SEQ ID NO:4 or other truncated, fusion or mutated C1orf54 polypeptides). The terms also refer to each of the polypeptides individually as well as jointly. For example, description of the preparation of, purification of, derivation of, administration of, compositions containing, treatment of a disease with etc., pertain to each polypeptide individually or jointly. A C1orf54 polypeptide variant is a protein that differs by at least one amino acid (e.g. deletion, substitution or addition) from the C1orf54 wild-type sequence having myokine activity, wherein the term "wild-type sequence" refers to a polypeptide or gene sequence available in at least one naturally occurring organism or a polypeptide or gene sequence that has not been changed, mutated, or otherwise manipulated by man. Such wild-type sequences can be isolated from nature or can be produced by recombinant or synthetic means.

The C1orf54 polypeptides described herein may be isolated from a variety of sources, such as from human tissues types, or prepared by recombinant or synthetic methods.

As used herein, the terms "polypeptide", "peptide" and "protein" are used interchangeably to describe a polymer of amino acid residues. The term "polypeptides" can mean, but is in no way limited to, recombinant full length, pro- and/or mature polypeptide forms as well as fragments or splice variants with biological activity, or recombinantly made truncations with biological activity derived from the full-length polypeptide. Recombinant forms of the polypeptides can be produced according to standard methods and protocols, which are well known to those of skill in the art, including for example, expression of recombinant proteins in prokaryotic and/or eukaryotic cells followed by one or more isolation and purification steps, and/or chemically synthesizing polypeptides or portions thereof using a peptide synthesizer. Furthermore, the term "polypeptide" applies to amino acid polymers in which one or more amino acid residue is an artificial chemical mimetic of a corresponding naturally occurring amino acid, as well as to naturally occurring amino acid polymers and non-naturally occurring amino acid polymers.

As used herein, the term "isolated polypeptide" is intended to refer to a polypeptide that is removed from its original or native environment (e.g. the natural environment if it is naturally occurring). For example, a naturally-occurring polynucleotide or polypeptide present in a living animal is not isolated, but the same polynucleotide or polypeptide, separated by human intervention from some or all of the co-existing materials in the natural system, is isolated. Such polynucleotides could be part of a vector and/or such polynucleotides or polypeptides could be part of a composition, and still be isolated in that such vector or composition is not part of the environment in which it is found in nature.

As used herein "conservatively modified variants" refers to individual substitutions, deletions or additions to a peptide, polypeptide, or protein sequence which alters, adds, substitutes and/or deletes a single amino acid or a small percentage of amino acids in the encoded sequence where the alteration results in the substitution of an amino acid with a chemically similar amino acid. Conservative substitution tables providing functionally similar amino acids are known in the art. Such conservatively modified variants are in addition to and do not exclude polymorphic variants, interspecies homologs, and alleles of the invention. Families of amino acid residues having similar side chains have been defined in the art. These families include amino acids with basic side chains (e.g. lysine, arginine, histidine), acidic side chains (e.g. aspartic acid, glutamic acid), uncharged polar side chains (e.g. glycine, asparagine, glutamine, serine, threonine, tyrosine, cysteine, tryptophan), nonpolar side chains (e.g. alanine, valine, leucine, isoleucine, proline, phenylalanine, methionine), beta-branched side chains (e.g. threonine, valine, isoleucine) and aromatic side chains (e.g. tyrosine, phenylalanine, tryptophan, histidine). Thus, a amino acid residue in a polypeptide or variant thereof can be preferably replaced with another amino acid residue from the same side chain family.

The term "sequence similarity" means that amino acids at the same position of the best sequence alignment are identical or similar, preferably identical. "Similar amino acids" possess similar characteristics, such as polarity, solubility, hydrophilicity, hydrophobicity, charge, or size.

The term "percent (%) amino acid sequence identity" with respect to the C1orf54 polypeptide and variant sequences as described herein is defined as the percentage of amino acid residues in a candidate sequence that are identical with the amino acid residues in the specific C1orf54 polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as EMBOSS Needle, BLAST, BLAST-2, ALIGN or Megalign (DNASTAR) software. Those skilled in the art can determine appropriate parameters for measuring alignment, including any algorithms needed to achieve maximal alignment over the full length of the sequences being compared. "Percent (%) amino acid sequence similarity" is determined by the same calculation used to determine % amino acid sequence identity, but including conservative amino acid substitutions in addition to identical amino acids in the computation.

The terms "identical" in the context of two or more nucleic acids or polypeptide sequences, refer to two or more sequences or subsequences that have the same sequences.

Analogous positions in two or more polypeptides can be determined by aligning the polypeptide sequences based on amino acid sequence or structural similarities. Such alignment tools can be, for example, obtained on the World Wide Web, e.g. ClustalW (www.ebi.ac.uk/clustalw) or Align (http://www.ebi.ac.uk/emboss/align/index.html) using standard settings, preferably for Align EMBOSS needle, Matrix:Blosum62, Gap Open 10.0, Gap Extend 0.5. Those skilled in the art understand that it may be necessary to introduce gaps in either sequence to produce a satisfactory alignment. Residues in two or more C1orf54 polypeptides or variants thereof are said to "correspond" if the residues are aligned in the best sequence alignment. The "best sequence alignment" between two polypeptides is defined as the alignment that produces the largest number of aligned identical residues.

As disclosed herein, the invention concerns an isolated C1orf54 polypeptide, preferably a human C1orf54 polypeptide, consisting of an amino acid sequence having at least about 80% amino acid sequence similarity, alternatively at least about 81% amino acid sequence similarity, alternatively at least about 82% amino acid sequence similarity, alternatively at least about 83% amino acid sequence similarity, alternatively at least about 84% amino acid sequence similarity, alternatively at least about 85% amino acid sequence similarity, alternatively at least about 86% amino acid sequence similarity, alternatively at least about 87% amino acid sequence similarity, alternatively at least about 88% amino acid sequence similarity, alternatively at least about 89% amino acid sequence similarity, alternatively at least about 90% amino acid sequence similarity, alternatively at least about 91% amino acid sequence similarity, alternatively at least about 92% amino acid sequence similarity, alternatively at least about 93% amino acid sequence similarity, alternatively at least about 94% amino acid sequence similarity, alternatively at least about 95% amino acid sequence similarity, alternatively at least about 96% amino acid sequence similarity, alternatively at least about 97% amino acid sequence similarity, alternatively at least about 98% amino acid sequence similarity, and alternatively at least about 99% amino acid sequence similarity, preferably 100% sequence identity, to a C1 orf54 polypeptide amino acid sequence over the entire length of the protein using the best sequence alignment and/or over the region of the best sequence alignment, wherein the best sequence alignment is obtainable with tools known in the art, e.g. Align, using standard settings, preferably EMBOSS::needle (Matrix: Blosum62, Gap Open 10.0, Gap Extend 0.5), with the amino acid sequence set forth herein as SEQ ID NO: 2.

"C1orf54 polypeptide variant" or "C1orf54 variant" means a biologically active C1orf54 polypeptide as defined herein having at least 80%, 81%, 82%, 83%, 84%, 85%, 86%, 87%, 88%, 89%, 90%, 91%, 92%, 93%, 94%, 95%, 96%, 97%, 98%, 99% amino acid sequence similarity, preferably 100% sequence identity, over the entire length of the protein using the best sequence alignment and/or over the region of the best sequence alignment, wherein the best sequence alignment is obtainable with art known tools, e.g. Align, using standard settings, preferably EMBOSS::needle (Matrix: Blosum62, Gap Open 10.0, Gap Extend 0.5), with a full-length C1orf54 polypeptide sequence, e.g. SEQ ID NO:2, or any other fragment of a full-length C1orf54 polypeptide sequence, e.g. SEQ ID NO:3 or SEQ ID NO:4 as described herein. A C1orf54 variant is also the C1orf54 precursor protein comprising a signal peptide sequence, e.g. a polypeptide comprising amino acid SEQ ID NO:1. A C1orf54 variant is also a fusion protein comprising a C1orf54 protein, e.g. a protein comprising a C1 orf54 protein fused to another, heterologous polypeptide or amino acid sequence, e.g. an immunoglobulin Fc region, human serum albumin (HSA) polypeptides, or conjugated to another moiety, e.g. polyethylene glycol (PEG), for example to increase the biological (e.g. serum) half-life of the C1 orf54 polypeptide or variant thereof. In one embodiment, such a C1orf54 variant comprises a fusion of C1orf54 polypeptide or variant thereof with a tag polypeptide. The tag is generally placed at the amino- or carboxyl terminus of the C1orf54 polypeptide. A C1orf54 variant is also a truncation of the full-length C1orf54 polypeptide or of the C1orf54 variant, wherein the truncation can be at the N-terminal of the polypeptide or at the C-terminal of the polypeptide or both at N-terminal of the polypeptide and at the C-terminal of the polypeptide. According to the present invention, polypeptides with SEQ ID NO:3 and SEQ ID NO:4 are such truncated variants of the full-length C1 orf54 polypeptide as set forth in SEQ ID NO:2. A variant also includes polypeptides with biological activity containing amino acid substitutions, modifications, additions and/or deletions at 1, 2, 3, 4, 5 or more amino acid positions, and have at least 80%, 81%, 82%, 83%, 84%, 85%, 86%, 87%, 88%, 89%, 90%, 91%, 92%, 93%, 94%, 95%, 96%, 97%, 98%, 99% sequence similarity, preferably 100% sequence identity, over the entire length of the fragment using the best sequence alignment and/or over the region of the best sequence alignment, wherein the best sequence alignment is obtainable with tools known in the art, e.g. Align, using standard settings, preferably EMBOSS::needle (Matrix: Blosum62, Gap Open 10.0, Gap Extend 0.5) with the amino acid sequence set forth in SEQ ID NO:2, 3, or 4. A C1orf54 variant as described herein retains its biological activity in the sense that such a protein can be considered as a functional equivalent of the full-length C1orf54 protein as set forth in SEQ ID NO:2, i.e. a variant retains the biological activity, e.g. of increasing muscle function as described herein, or a variant exceeds at least one of the biological activities observed for the full-length C1orf54 polypeptide as set forth in SEQ ID NO: 2,as assessed in a suitable biological system, e.g. in vitro or *in vivo,* as described herein. A C1orf54 variant is also the isolated polypeptide comprising any of the amino acid sequence SEQ ID NO:5-9 or SEQ ID NO:10-14.

Furthermore, functional equivalents with regard to the C1orf54 polypeptide are understood as C1orf54 polypeptides or variants thereof comprising at least one natural or artificial mutation. Mutations can be insertions, deletions or substitutions of one or more amino acids that do not diminish the biological activity of the C1orf54 polypeptide.

In case of fusion proteins as described above, the sequence identity or sequence similarity shall be defined only on the basis of the C1orf54 part of such a fusion protein, i.e. additional amino acids not derived from C1orf54 polypeptide or variants thereof will not be considered in such an alignment, i.e. are excluded from the calculation of the alignment score. In case of truncated proteins as described above, the sequence identity or sequence similarity shall be defined only on the basis of the truncated C1orf54 polypeptide or variant thereof. Variants may exhibit amino acid sequences that are at least 80%, 81%, 82%, 83%, 84%, 84%, 85%, 86%, 87%, 88%, 89%, 90%, 91%, 92%, 93%, 94%, 95%, 96%, 97%, 98%, 99% identical to the fused or truncated C1orf54 polypeptide variant, e.g. to C1orf54 polypeptide variant with SEQ ID NO:3 or SEQ ID NO:4.

Methods of alignment of sequences for comparison are known in the art. Optimal alignment of sequences for comparison can be conducted, e.g. by the local homology algorithm of Smith and Waterman (1970) Adv. Appl. Math. 2:482c, by the homology alignment algorithm of Needleman and Wunsch (1970) J. Mol. Biol. 48:443, by the search for similarity method of Pearson and Lipman (1988) Proc. Nat'l. Acad. Sci. USA 85:2444, by computerized implementations of these algorithms (GAP, BESTFIT, FASTA, and TFASTA in the Wisconsin Genetics Software Package, Genetics Computer Group, 575 Science Dr., Madison, Wl), or by manual alignment and visual inspection (see, e.g. Ausubel et al., Current Protocols in Molecular Biology (1995 supplement)).

A preferred algorithm for sequence comparison is EMBOSS needle, which reads two input sequences and writes their optimal global sequence alignment to file. The program uses the Needleman-Wunsch alignment algorithm to find the optimum alignment (including gaps) of two sequences along their entire length. The standard settings for EMBOSS needle are BLOSUM62 scoring matrix with penalties of 10.0 for Gap Open 10.0 and 0.5 for Gap Extend.

C1orf54 polypeptide is part of a complex system that muscle cells use in order to communicate with their physiologic environment and it is therefore classed as a myokine.

As used herein, the term "myokine" refers to cytokines, peptides or polypeptides, proteoglycan peptides, bioactive lipids, second messengers, etc. produced, expressed, and released by muscle cells.

As used herein, the term "myokine activity" refers to autocrine, paracrine and/or endocrine effects produced by a myokine, or myokine signal, on a subject, cell, ortissue, e.g. adipose tissue, brain, pancreas, gut, liver, immune cells, bone, liver and tumor cells. Myokine activity may be inhibitory or stimulatory. Such "myokine activity" may result in muscle hypertrophy, increased muscle regeneration, increased muscle strength (force generating capacity), proliferation of primary human skeletal muscle cells, increased muscle innervation, effects on extracellular matrix remodeling, modulation of bone metabolism (e.g. bone mass, bone density, osteogenesis, bone mineralization, bone healing), modulation of adipose metabolism (e.g. increased lipolysis, decreased lipoprotein lipase activity, increased browning of adipose tissue, increased thermogenesis, decreased adipose tissue mass, increased fatty acid oxidation, decreased levels of circulating and/or hepatic triglycerides, decreased total body fat, decreased visceral fat), modulation of immune cell responses, regulation of immunomodulatory processes, modulation of inflammatory processes, modulation of responses of immune cells, reduced inflammation, stimulation of energy expenditure, modulation of whole-body metabolism, modulation of whole-body insulin sensitivity, regulation of glucose metabolism (e.g. increased glucose uptake, increased glycogen synthesis, increased glucose oxidation, improved glucose tolerance, improved insulin sensitivity), promotion of pancreatic beta-cell viability, alpha-cell hyperplasia, systemic cardiovascular adaptations (including increased cardiac output, enhanced blood volume), increased angiogenesis (e.g. vascularization, capillarization of or near skeletal muscle cells, improved oxygen supply and utilization, increased vascular repair processes, decreased atherogenesis, control of blood pressure), cardioprotection, improved cognitive function and memory, decreased stress and depression, brain neurogenesis, cancer protection (e.g. through increased apoptosis and mobilization of immune effector cells), and anti-aging effects. As used herein, said myokine activity preferably leads to an increase in muscle function.

As used herein, the term "increase in muscle function" or "increased muscle function" or "muscle function increasing activity" (and the like) is intended to refer to an activity that leads at least one or more of i) an increase in muscle strength, ii) an increase in muscle precursor cell proliferation, iii) improved muscle regeneration activity or iv) increased muscle fatigability resistance. The terms "increase(d)" or "improved" refer to a condition under which a parameter to be measured *in vivo* or in vitro (e.g. muscle strength, muscle precursor cell proliferation, muscle regeneration activity, satellite cell proliferation assay or muscle fatigability resistance) is greater at a second point in time compared to a first point in time. Said parameters can be assessed using a suitable *in vitro* and/or *in vivo* assay, e.g. as described herein, e.g. human myoblast *in vitro* proliferation assay or mouse satellite cell proliferation *in vivo* or methods to evaluate muscle strength.

As used herein, the term "muscle strength" (and the like) refers to the maximum amount of force a muscle, or muscle groups in sum, can generate. Muscle strength may be evaluated by a variety of methods such as grip strength, one repetition maximum strength test, time-dependent tests of muscle endurance, time-dependent tests of muscle fatigue, or time-dependent tests of muscle endurance and fatigue, and so forth. Muscle strength may be assessed by dynamic (e.g. 30 second chair stand test, stair climb test, knee flexion/extension) or static (e.g. hand grip dynamometry, isometric knee extension/flexion) movements across single or multiple joints and respiratory and other muscles (e.g. pulmonary function tests).

As used herein, the term "increased muscle strength" (and the like) in a subject is also intended to refer to an increase in muscle strength and/or physical performance compared to before treatment, and as measured after at least 2 weeks, at least 4 weeks, after at least 8 weeks, after at least 12 weeks, after at least 16 weeks, after at least 20 weeks, after at least 24 weeks, after at least 28 weeks, after at least 32 weeks, after at least 36 weeks, after at least 40 weeks, after at least 44 weeks, after at least 48 weeks, after at least 52 weeks or more of treatment.

Physical performance may be assessed by Short Physical Performance Battery (SPPB), gait speed measurement, timed get-up-and-go test (TGUG), stair climb power test (SCPT) 400 m walk test and/or 6-minute walk test for distance (6MWD).

As used herein, the term "regulate" is used to describe that the level or activity of a function is greater than, equal to or less than that observed in the absence of an agent provided by the invention.

The term "subject" or "patient" as used herein is intended to include humans and animals, which are capable of suffering from or afflicted with a muscle-related disorder or disease or would otherwise benefit from increase in muscle function. Examples of subjects include mammals, e.g. humans, dogs, cows, horses, pigs, sheep, goats, cats, and transgenic non-human animals. In one embodiment, the subject is a human, e.g. a human suffering from, at risk of suffering from, or potentially capable of suffering from muscle-related disorder. In a preferred embodiment, the subject is a human subject, e.g. a human patient having a muscle-related disorder. In another embodiment the subject is an animal, e.g. a cow or pig which is treated in accordance with the herein disclosed method (e.g. administration of C1 orf54) in order to increase lean body mass or the muscle function of said animal.

As used herein, a subject is "in need of' a treatment if such subject (patient) would benefit biologically, medically or in quality of life from such treatment.

As used herein, the term "muscle mass" refers to the amount or size of muscle or muscle groups, as expressed by muscle weight, mass, area, or volume. Muscle mass may also be expressed as total lean body mass, lean body mass of a body compartment such as the leg, or cross-sectional area of a leg or arm compartment. The volume or mass of the muscle can be determined using any known or otherwise effective technique that provides muscle area, volume, or mass, such as Dual energy X-ray absorptiometry (DXA), Bioimpedance analysis (BIA), computed tomography (CT) scan, ultrasound, magnetic resonance imaging (MRI) or total or partial body potassium per fat-free soft tissue (TBK or PBK).

As used herein, the term "satellite cells" is intended to refer to small mononuclear stem cells that are naturally located underneath the basal lamina of the myofiber, and that are capable of self-renewal and of forming new skeletal muscle cells (i.e. myogenic). The most definitive marker of satellite cells is Pax7, which is present in all satellite cells of post-natal muscles and expressed in proliferating myoblasts until they begin to differentiate.

As used herein, the terms "regenerative capacity of muscle", "muscle regeneration activity" or "muscle regeneration improving activity" (and the like) is intended to refer to the process of re-growth or repair of muscle cells in order to increase, recover or maintain the total number of viable or functional myocytes and provide replacement of compromised muscle fibers in response to damage such as tissue loss following disease or injury. In response to this damage, satellite cells are activated and become committed myoblasts, which then begin to proliferate to expand the population of progenitor for repair (see e.g. Kuang and Rudnicki (2008) Trends Mol Med 14, 82-91). Finally, proliferating myoblasts withdraw from the cell cycle and undergo terminal differentiation. They fuse with each other to form renascent myofibers, replacing the damaged and dead ones. The terms "regenerative capacity of muscle" or "muscle regeneration activity" (and the like) also include the process of satellite cell-mediated growth (re-enter the cell cycle, proliferate and fuse into existing muscle fibres or form new muscle fibre), syncytial myofiber growth or myoblast-dependent *de novo* myofiber differentiation, whereby myofiber growth may also be myoblast-dependent and *de novo* myofiber differentiation may be satellite cell-dependent, whereby myofibres fuse into existing muscle fibres and/or increase the size of regenerating myofibers and/or form new muscle fibres, thereby promoting skeletal muscle regeneration.

As used herein, the term "muscle-related disorder" is intended to refer to a disease or disorder resulting in muscle dysfunction (e.g. reduced muscle strength or power, reduced regenerative capacity and/or reduced endurance), such that the muscle is unable to perform its function at a level needed for a person to maintain normal daily independence. A subject may be impaired to perform their physiological tasks adequately. Such muscle dysfunction may result in physical disability, impaired mobility, loss of functional independence, frequent falling, fear of falling, injurious falling, muscle wasting, impaired and unsteady gait, adverse gait mechanics, scoliosis, trouble running and jumping, difficulty lifting objects, myotonia, drooping eyelids, muscle hypertrophy, muscle hypotrophy or atrophy, respiratory difficulty, inability to walk, learning disabilities, poor balance, difficulty getting up from a lying or sitting position, use of the Gower's maneuver to stand, and/or reduced endurance.

As used herein, the term "muscle loss", is intended to refer to a condition of deterioration of muscle quantity and quality. Non-limiting symptoms of muscle loss can be loss or reduction of muscle mass, loss or reduction of lean muscle, loss or reduction of muscle weight, loss or reduction of muscle circumference, loss or reduction of muscle function, loss or reduction of muscle strength, loss or reduction of mobility, or any combination thereof. For example, muscle loss can be caused by aging, disease (for example cancer and liver diseases), inactivity, injury (for example liver transplantation), or any combination thereof. As used herein, the term "muscle loss" encompasses the natural process of muscle ageing, as well as congenital pathologies and acquired pathologies. Muscle ageing results in progressive skeletal muscle loss, and it includes, without limitation, sarcopenia. The term "muscle loss" also encompasses any congenital or acquired muscular degeneration, weakness, dysfunction and/or loss affecting the skeletal muscle(s). Congenital pathologies, without limitation, include myopathies (e.g. Duchenne muscular dystrophy (DMD), Becker muscular dystrophy, Congenital muscular dystrophy, Limb Girdle muscular dystrophy (LGMD), facioscapulohumeral muscular dystrophy, myotonic muscular dystrophy, oculopharyngeal muscular dystrophy, distal muscular dystrophy, and Emery-Dreifuss muscular dystrophy). In contrast, acquired pathologies affecting the skeletal muscle(s) are not inherited and can be induced by inflammation (e.g. inflammatory myopathies), drugs (e.g. drug-induced myopathy), trauma or injury (e.g. trauma-induced myopathy or surgery-induced myopathy), connective tissue and muscle ischemia (e.g. Buerger disease), cancer (e.g. cancer-induced sarcopenia) or even diet (e.g. diet-induced myopathy). In some embodiments, the disclosed compositions, uses, kits, methods and compounds reduce, arrest or reverse muscle loss in a patient.

As used herein, the term "secondary muscle loss", is intended to refer to a condition of deterioration of muscle quantity and quality resulting from injury, illness or trauma. These include, but are not limited to, stroke, motor vehicle accidents, severe burns, combat injuries, chronic obstructive pulmonary disease (COPD), diabetes, cancer, activities associated with surgery, inability to move muscles and joints for an extended period of time (e.g. bedrest, casting) and other conditions that create a negative nitrogen balance in the body. Disuse of greater than 10 days bedrest has long been recognized to result in loss of skeletal muscle, strength and patient function, but it is now also understood that considerable muscle loss and atrophy occurs already after short-term disuse (less than 10 days) (Dirks et al. (2016) Diabetes 65, 2862-2875, Wall et al. (2013) Ageing Res Rev 12, 898-906*).* Therefore, the term "secondary muscle loss" encompasses muscle loss occurring after short-term disuse as well as long-term disuse. In some embodiments, the disclosed compositions, uses, kits, methods and compounds reduce, arrest or reverse secondary muscle loss in a patient.

As used herein, the term "sarcopenia" refers to a syndrome characterized by progressive and generalized loss of skeletal muscle mass, strength and impaired functional capacity seen in older adults as defined in *Sarcopenia: European consensus on definition and diagnosis* (Cruz-Jentoft et al. (2010) Age Ageing 39, 412-423) and by the International Working Group on Sarcopenia (Fielding et al. (2011) J Am Med Dir Assoc 12, 249-256). In addition, sarcopenia is used to describe the loss of skeletal muscle mass and function associated with diseases, surgery and other conditions (cancer (Collins et al (2014) BMJ Open;4:e003697. doi:10.1136/bmjopen-2013-003697), cirrhosis (Georgiou et al (2018) Clin NutrESPEN. 24:185-186), chronic obstructive pulmonary diseases (Jones et al (2015) Thorax. 70:213-8), peripheral arterial disease (Addison et al (2018) Arch Phys Med Rehabil. 99(4):623-628), post stroke (Ryan et al (2017) Arch Phys Med Rehabil. 98(3):495-499), and heart failure (Saitoh et al (2018) Expert Rev Cardiovasc Ther. 16(2):133-142), and in those undergoing organ transplant (Meek and Madill (2017) Clin Nutr ESPEN. 22:76-80) and recovering from hip fracture (Landi et al (2017) Osteoporos Int. 28:1569-1576. doi: 10.1007/s00198-017-3929-z).*Generally,* a diagnosis of sarcopenia requires the presence of low muscle mass and at least one of low muscle strength and low physical performance. The term "sarcopenia" includes primary sarcopenia (age-related sarcopenia) and secondary sarcopenia where the muscle and functional loss are a consequence of another condition (including activity-related sarcopenia, such as resulting from bed rest, inactivity, sedentary lifestyle, and deconditioning; disease-related sarcopenia associated with advanced organ failure of heart, lung, liver, kidney and/or brain, inflammatory disease, malignancy or endocrine disease and nutrition-related sarcopenia resulting from inadequate dietary intake of energy and/or protein, such as with malabsorption, gastrointestinal disorders or use of medications that cause anorexia). In some embodiments, the disclosed compositions, uses, kits, methods and compounds reduce, arrest or reverse sarcopenia in a patient.

As used herein, the term "cachexia" refers to wasting that is characterized by progressive weight loss, muscle wasting/loss and fatigue, due to the loss of skeletal muscle with or without loss of adipose tissue as defined by *Special Interest Groups (SIG) "cachexia-anorexia in chronic wasting diseases*" *and* "*nutrition in geriatrics*" (Muscaritoli et al. (2010) Clin Nutr 29, 154-159). Cachexia is frequently associated with inflammation, insulin resistance, anorexia and increased breakdown of muscle proteins due to severe chronic illness, such as cancer, chronic heart failure, chronic cardiomyopathy, chronic renal failure, end-stage renal disease, chronic obstructive pulmonary disease (COPD), liver failure, liver cirrhosis, acquired immunodeficiency syndrome (AIDS), severe burn injury and rheumatoid arthritis. In some embodiments, the disclosed compositions, uses, kits, methods and compounds reduce, arrest or reverse cachexia in a patient.

As used herein, the term "muscle weakness" includes the loss of muscle strength seen following a trauma such as surgery and acute or long-term injury, disuse, e.g. following a stroke, or high levels of inflammation or other conditions resulting in a decrease in force around a joint caused by the lower force generating capacity of individual or groups of muscles regardless of the contraction intensity. Post-surgical muscle weakness refers to a reduction in the strength of one or more muscles following surgical procedure. Weakness may be generalized (i.e. total body weakness) or localized to a specific area, side of the body, limb, or muscle. Post-traumatic muscle weakness is characterized by a reduction in the strength of one or more muscles following a traumatic episode (e.g. bodily injury, surgery or stroke). In some embodiments, the disclosed compositions, uses, kits, methods and compounds reduce, arrest or reverse muscle weakness in a patient.

As used herein, the term "administering" is intended to refer to a method of giving a dosage of a pharmaceutically active ingredient, e.g. a C1 orf54 polypeptide or variant thereof as described herein, to a subject in need thereof.

The term "pharmaceutically acceptable" as used herein refers to those compounds, materials, compositions and/or dosage forms, which are, within the scope of sound medical judgment, suitable for contact with the tissues of a subject, e.g. a mammal or human, without excessive toxicity, irritation, allergic response and other problems or complications commensurate with a reasonable benefit/risk ratio.

As used herein, the term "pharmaceutically acceptable carrier, diluent or excipient" or "carrier, diluent or excipient" refers to a substance useful in the preparation or use of a pharmaceutical composition, which enhance or stabilize the composition, or can be used to facilitate the preparation of the composition. Pharmaceutically acceptable carriers include solvents, dispersion media, coatings, antibacterial and antifungal agents, isotonic and absorption delaying agents, and the like, and includes, for example, suitable diluents, surfactants, antioxidants, preservatives, buffering agents, bulking agents, cryoprotectants, lyoprotectants, salts, drug stabilizers, binders, excipients, disintegration agents, lubricants, wetting agents, sweetening agents, flavoring agents, dyes, and combinations thereof, solvents, dispersion media, coatings, antibacterial and antifungal agents, isotonic and absorption delaying agents, and the like that are physiologically compatible and that do not produce an adverse, allergic or other untoward reaction when administered to an animal, or a human, as appropriate (see, for example, Remington The Science and Practice of Pharmacy, 22nd Ed. Pharmaceutical Press, 2013, pp. 1049-1070). Except insofar as any conventional media or agent is incompatible with the active compound, use thereof in the compositions described herein is contemplated.

The term "pharmaceutical composition" as used herein, refers to a C1 orf54 polypeptide or variant thereof together with at least one pharmaceutically acceptable carrier, diluent or excipient in a form suitable for administration to the physical location most suitable for their desired activity, e.g. systemic administration. It is intended that the term "pharmaceutical composition" refers to a mixture or solution containing at least one therapeutic agent, preferably an C1orf54 polypeptide or variant thereof, to be administered to a subject, e.g. a mammal or human, in order or treat a particular disease or condition affecting the subject. If not indicated otherwise, these are prepared in a manner known *per se*, for example by means of various conventional mixing, comminution, direct compression, granulating, sugar-coating, dissolving, lyophilizing processes, or fabrication techniques readily apparent to those skilled in the art. It will be appreciated that the unit content of an individual dose of each dosage form need not in itself constitute an effective amount since the necessary effective amount may be reached by administration of a plurality of dosage units. The amount of active agent which can be combined with a carrier material to produce a single dosage form will vary depending upon the subject being treated, and the particular mode of administration. The amount of active agent which can be combined with a carrier material to produce a single dosage form will generally be that amount of the composition which produces a therapeutic effect. Generally, out of one hundred percent, this amount may range from about 0.01 per cent to about ninety-nine percent of active agent, from about 0.1 per cent to about 70 per cent, or from about 1 percent to about 30 percent of active agent in combination with a pharmaceutically acceptable carrier.

The pharmaceutical compositions of the invention may include a "therapeutically effective amount" or "effective amount" of a C1orf54 polypeptide or variant thereof described herein. As used herein, the terms "effective amount", "effective dose", "pharmaceutically effective amount", "pharmaceutically effective dose", "therapeutically effective amount" or "therapeutically effective dose" (and the like) are used interchangeably and are intended to refer to an amount/dose of the C1orf54 polypeptide or variant thereof described herein, which is sufficient, at dosages and for periods of time necessary, to provide an observable or clinically significant improvement over the baseline of clinically observable signs and symptoms of muscle-related disease or disorder and/or to prevent, inhibit the occurrence, ameliorate, delay, alleviate, arrest, reverse or treat one or more symptoms associated with a muscle-related disease or disorder. The effective amount depends on the type of disease, the composition used, the route of administration, the type of mammal being treated, the physical characteristics of the specific mammal under consideration, concurrent medication, and other factors such as the disease state, age, sex, and weight of the individual which those skilled in the medical arts will recognize. A therapeutically effective amount is also one in which any toxic or detrimental effects of the therapeutic agents are outweighed by therapeutically beneficial effects. Toxicity and therapeutic efficacy of such compounds can be determined by standard pharmaceutical procedures in cell cultures or experimental animals, e.g. for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Compounds that exhibit large therapeutic indices are preferred. While compounds that exhibit toxic side effects may be used, care should be taken to design a delivery system that targets such compounds to the site of affected tissue in order to minimize potential damage to uninfected cells and, thereby, reduce side effects. The data obtained from the cell culture assays and animal studies can be used in formulating a range of dosage for use in humans. The dosage of such compounds lies preferably within a range of circulating concentrations that include the ED50 with little or no toxicity. The dosage may vary within this range depending upon the dosage form employed and the route of administration utilized. For any compound used in the method of the invention, the therapeutically effective dose can be estimated initially from cell culture assays. A dose may be formulated in animal models to achieve a circulating plasma concentration range that includes the IC50 (i.e. the concentration of the test compound which achieves a half-maximal inhibition of symptoms) as determined in cell culture. Such information can be used to more accurately determine useful doses in humans. Levels in plasma may be measured, for example, by high performance liquid chromatography. In another embodiment, the term "a therapeutically effective amount" refers to the amount of the C1orf54 polypeptide or variant thereof of the present invention that, when administered to a cell, or a tissue, or a non-cellular biological material, or a medium, is effective to at least partially act as a myokine on the cell, tissue or non-cellular biological material.

As used herein, the term "prevent", "preventing" or "prevention" in connection to a disease or disorder refers to the prophylactic treatment of a subject who is at risk of developing a condition (e.g. a specific disease or disorder or clinical symptom thereof) resulting in a decrease in the probability that the subject will develop the condition. In some embodiments, the disclosed compositions, uses, kits, methods and compounds are used in prophylactic treatment in a patient.

As used herein, the term "treat", "treating" or "treatment" in connection to a muscle-related disorder refers in one embodiment, to ameliorating the disease or disorder (i.e., slowing or arresting or reducing the development of the disease or at least one of the clinical symptoms thereof). In another embodiment "treat", "treating" or "treatment" refers to alleviating or ameliorating at least one physical parameter including those which may not be discernible by the patient. In yet another embodiment, "treat", "treating" or "treatment" refers to modulating the disease or disorder, either physically, (e.g. stabilization of a discernible symptom), physiologically, (e.g. stabilization of a physical parameter), or both. In yet another embodiment, "treat", "treating" or "treatment" refers to delaying the onset or development or progression of the muscle-related disorder.

All methods described herein can be performed in any suitable order unless otherwise indicated herein or otherwise clearly contradicted by context. The use of any and all examples, or exemplary language (e.g. "such as") provided herein is intended merely to better illuminate the invention and does not pose a limitation on the scope of the invention otherwise claimed.

### DETAILED DESCRIPTION OF THE INVENTION

The present invention is based, in part, on the finding that C1orf54 polypeptide or variants thereof have myokine activity. It has been discovered that the levels of expression of *C1ORF54* mRNA are increased in skeletal muscle in response to exercise in subjects.

Accordingly, the present invention provides C1orf54 variants thereof having myokine activity. The present invention also provides C1orf54 polypeptide or variants thereof for use as a medicament. The present invention also provides C1orf54 polypeptide or variants thereof for use in therapy or for the use in the preparation of a medicament. The present invention also provides pharmaceutical compositions comprising C1orf54 polypeptide or variants thereof for use in the treatment, prevention or amelioration of a muscle-related disorder. Consequently, highly desirable C1orf54 polypeptides and variants thereof having myokine activity and novel therapeutic approaches are provided by the present invention.

Further provided are kits comprising a C1orf54 polypeptide or variants thereof (e.g. in liquid or lyophilized form) as described herein or a pharmaceutical composition comprising the C1 orf54 polypeptide or variants thereof as described herein.

The invention further provides methods of treating, preventing, or ameliorating muscle-related disorders, comprising administering to a subject in need thereof an effective amount of an isolated C1orf54 polypeptide or variants thereof having myokine activity.

In one embodiment, the present invention provides a polynucleotide molecule encoding an amino acid sequence having at least 90% or at least 95% sequence identity to an amino acid sequence selected from SEQ ID NO:3 or SEQ ID NO:4, wherein said amino acid sequence is encoding a C1orf54 variant with myokine activity.

Also provided is a method of producing a recombinant polypeptide whose amino acid sequence is at least 80%, 85%, 90%, 95%, 97%, 99%, or 100% identical to the amino acid sequence of SEQ ID NO:2, 3, or 4; or an amino acid sequence encoded by a nucleic acid having at least 80%, 85%, 90%, 95%, 97%, 99%, or 100% sequence identity to the polynucleotide sequence of SEQ ID NO:16, 17, or 18, comprising: a) providing a nucleic acid sequence operatively linked to a heterologous regulatory sequence; and b) expressing the nucleic acid of (a) under conditions that allow expression of the polypeptide, thereby producing a recombinant polypeptide. Also provided are vectors comprising a polynucleotide selected from SEQ ID NO:16, 17, or 18 encoding the C1orf54 polypeptides and variants thereof produced, e.g. C1orf54 polypeptides and variants thereof comprising amino acid SEQ ID NO:2, 3, or 4, as described in the method above operably linked to a heterologous promoter. Also provided is an isolated host cell comprising one or more of said vectors. Also provided is a process for producing a C1orf 54 polypeptide or variant thereof according to the method above, comprising (a) culturing the host cell comprising one or more of said vectors; and (b) recovering said C1orf54 polypeptide or variant thereof.

Alternatively, provided is a method of producing a recombinant polypeptide whose amino acid sequence is at least 80%, 85%, 90%, 95%, 97%, 99%, or 100% identical to the amino acid sequence of SEQ ID NO:10-14; or an amino acid sequence encoded by a nucleic acid having at least 80%, 85%, 90%, 95%, 97%, 99%, or 100% sequence identity to the polynucleotide sequence of SEQ ID NO:19-23, comprising: a) providing a nucleic acid sequence operatively linked to a heterologous regulatory sequence; and b) expressing the nucleic acid of (a) under conditions that allow expression of the polypeptide, thereby producing a recombinant polypeptide. Also provided are vectors comprising a polynucleotide selected from SEQ ID NO:19-23 encoding the C1orf54 polypeptides and variants thereof produced, e.g. C1orf54 polypeptides and variants thereof comprising amino acid SEQ ID NO:10-14, as described operably linked to a heterologous promoter. Also provided is an isolated host cell comprising one or more of said vectors. Also provided is a process for producing a C1orf 54 polypeptide or variant thereof according to the method above, comprising (a) culturing the host cell comprising one or more of said vectors; and (b) recovering said C1orf54 polypeptide or variant thereof.

Other investigators previously described the cloning and isolation of polynucleotides encoding C1orf54 (Gregory et al. (2006) Nature 441, 315-321, Hartley et al. (2000) Genome Res 10, 1788-1795, Strausberg et al. (2002) Proc Natl Acad Sci USA 99, 16899-16903) and the fact that the encoded polypeptide is secreted (WO1998/42738, WO1999/18127, EP1104808).

A variety of C1orf54 polypeptides and variants thereof can be used according to the present invention. The present invention contemplates use of various C1orf54 polypeptides and variants thereof having myokine activity. In some embodiments, the invention provides for use of full-length C1orf54 polypeptide amino acid sequences or variants thereof. In some embodiments, the invention provides for C1orf54 polypeptides comprising a portion (not the full-length sequence) of the C1orf54 sequence, or a variant thereof, that retains myokine activity. Thus, in some embodiments, the C1 orf54 polypeptides of the invention comprise at least a fragment (e.g. at least 60, at least 65, at least 70, at least 75, at least 80, at least 85, at least 90, at least 95, at least 100, at least 105, at least 110 contiguous amino acids) of the human (e.g. SEQ ID NO:2) C1orf54 protein sequence, having myokine activity.

In some embodiments, the C1orf54 proteins of the invention comprise at least a fragment (e.g. at least 60, at least 65, at least 70, at least 75, at least 80, at least 85, at least 90, at least 95, at least 100, at least 105, at least 110 contiguous amino acids) of the native mouse (e.g. SEQ ID NO: 24), canine (e.g. SEQ ID NO:32), bovine (e.g. SEQ ID NO:33) or equine (e.g. SEQ ID NO:31) C1orf54 polypeptide sequence.

In some embodiments, the invention provides C1orf54 polypeptide variants (including but not limited to any of SEQ ID NOs:1-14) lacking at least a portion of the C-terminal sequence, e.g. lacking at least 10, 20, 30, 40, 50 amino acids from the C-terminus, e.g. 10, 20, 30, 40, 50 amino acids.

In some embodiments, the invention provides C1orf54 polypeptide variants (including but not limited to any of SEQ ID NOs:1-14) lacking at least a portion of the N-terminal sequence, e.g. lacking at least 10, 20, 30, 40, 50 amino acids from the N-terminus, e.g. 10, 20, 30, 40, 50 amino acids.

The C1 orf54 polypeptides of the invention encompass variants and truncations of naturally-occurring C1orf54 polypeptides as well as variants and truncations of C1 orf54 polypeptide variants described herein having myokine activity. The invention encompasses the use of C1orf54 polypeptides variants and truncations of naturally-occurring C1 orf54 polypeptides having myokine activity in methods of the invention. Active variants having myokine activity can be identified in any number of ways known to those of skill in the art, e.g. as described herein. In some embodiments, amino acid alignments of active proteins can be established to identify those positions that are invariant or that are include conserved amino acid changes.

Accordingly, one aspect of the invention provides an isolated polypeptide consisting of an amino acid sequence having at least 95%, at least 97% or at least 99% amino acid sequence identity to an amino acid sequence of SEQ ID NO:3, wherein the polypeptide has myokine activity.

Another aspect of the invention provides an isolated polypeptide consisting of an amino acid sequence having at least 95%, at least 97% or at least 99% amino acid sequence identity to an amino acid sequence of SEQ ID NO:4, wherein the polypeptide has myokine activity.

In one embodiment, provided is an isolated polypeptide comprising or consisting of an amino acid sequence selected from the group of SEQ ID NO:3 and SEQ ID NO:4, and wherein one, two, three, four or five amino acids have been modified, deleted or substituted within the sequence of any one of SEQ ID NO:3 or SEQ ID NO:4 and wherein the polypeptide has myokine activity.

In another embodiment, the invention provides an isolated polypeptide consisting of an amino acid sequence of SEQ ID NO:3 and wherein the polypeptide has myokine activity.

In another embodiment, the invention provides an isolated polypeptide consisting of an amino acid sequence of SEQ ID NO:4 and wherein the polypeptide has myokine activity.

The C1 orf54 polypeptides of the invention can include also non-native C1orf54 protein flanking sequences. For example, a portion of the C1orf54 polypeptide with myokine activity can be fused to one or more heterologous amino acids to form a fusion protein. Fusion partner sequences can include, but are not limited to, amino acid tags, non-L (e.g. D-) amino acids or other amino acid mimetics to extend *in vivo* half-life and/or protease resistance, targeting sequences or other sequences, e.g. the human immunoglobulin Fc fragment. In one embodiment the invention provides a C1orf54 polypeptide fused to one or more heterologous amino acids, a peptide or protein, wherein the heterologous amino acids, peptide or fusion protein partner can include amino acid tags, *in vivo* half-life extenders, targeting sequences, a human immunoglobulin Fc fragment.

In a further embodiment, the isolated C1orf 54 polypeptide according to the invention, e.g. an isolated C1orf54 polypeptide consisting of an amino acid sequence selected from the group of SEQ ID NO:3 and SEQ ID NO:4, has myokine activity, wherein said myokine activity comprises muscle function increasing activity.

In another embodiment, the isolated C1orf54 polypeptide according to the invention, e.g. an isolated C1orf54 polypeptide consisting of an amino acid sequence selected from the group of SEQ ID NO:3 and SEQ ID NO:4, has myokine activity, wherein said myokine activity comprises muscle strength increasing activity.

In yet another embodiment, the isolated polypeptide according to the invention, e.g. an isolated C1orf54 polypeptide consisting of an amino acid sequence selected from the group of SEQ ID NO:3 and SEQ ID NO:4, has myokine activity, wherein said myokine activity comprises muscle regeneration improving activity.

### PHARMACEUTICAL COMPOSITIONS

Provided herein are pharmaceutical compositions comprising an isolated C1orf54 polypeptide or variants thereof having myokine activity, e.g. C1orf54 polypeptide or variants thereof comprising an amino acid sequence of SEQ ID NO:2, 3, or 4, formulated together with a pharmaceutically acceptable carrier, excipient or diluent. The compositions can additionally contain one or more other therapeutic agents that are suitable for treating a medical condition.

A pharmaceutical composition described herein can be administered by a variety of methods known in the art. The route and/or mode of administration can vary depending upon the desired results. Depending on the route of administration, the C1orf54 polypeptides or variants thereof having myokine activity may be coated in a material to protect the compound from the action of acids and other natural conditions that may inactivate the compound. Thus, in another aspect, the present invention provides a composition, e.g. a pharmaceutical composition, containing the C1orf54 polypeptides or variants thereof disclosed herein and at least one pharmaceutically acceptable carrier, diluent or excipient.

Accordingly, provided is a pharmaceutical composition comprising a pharmaceutically effective amount of at least one polypeptide selected from:
i. a polypeptide consisting of amino acid sequence SEQ ID NO:2, 3, or 4;
ii. a polypeptide comprising an amino acid sequence of SEQ ID NO:2, 3, or 4;
   a polypeptide consisting of an amino acid sequence having at least 95% identity to SEQ ID NOs:2, 3, or 4;
iv. a polypeptide comprising an amino acid sequence having at least 95% identity to SEQ ID NOs:2, 3, or 4; and
v. a polypeptide comprising or consisting of an amino acid sequence, wherein one, two, three, four or five amino acids have been modified, deleted, added and/or substituted within the sequence of any one of SEQ ID NOs:2, 3, or 4; and
wherein said polypeptide has myokine activity; and at least one pharmaceutically acceptable carrier, diluent or excipient.

Also provided is a pharmaceutical composition comprising at least one polypeptide selected from:
i. a polypeptide consisting of amino acid sequence SEQ ID NO:2, 3, or 4;
ii. a polypeptide comprising an amino acid sequence of SEQ ID NO:2, 3, or 4;
iii. a polypeptide consisting of an amino acid sequence having at least 95% identity to SEQ ID NOs:2, 3, or 4;
iv. a polypeptide comprising an amino acid sequence having at least 95% identity to SEQ ID NOs:2, 3, or 4;
v.a polypeptide comprising or consisting of an amino acid sequence, wherein one, two, three, four or five amino acids have been modified, deleted, added and/or substituted within the sequence of any one of SEQ ID NOs:2, 3, or 4; and
wherein said polypeptide has myokine activity; and at least one pharmaceutically acceptable carrier, diluent or excipient. In another embodiment, said pharmaceutical composition is for use in the treatment of a muscle-related disorder. In one embodiment, said myokine activity comprises muscle function increasing activity. In one embodiment, said muscle function increasing activity is determined by the assays described herein. In another embodiment, said myokine activity may comprise muscle strength increasing activity. In another embodiment, said muscle strength increasing activity is determined by the assay described herein. In yet another embodiment said myokine activity may comprise muscle regeneration improving activity. In yet another embodiment said muscle regeneration improving activity is determined by the assays described herein. In another embodiment, said myokine activity may comprise muscle strength increasing activity and muscle regeneration improving activity. In one embodiment, the pharmaceutical compositions provided herein are for use in the treatment of a muscle-related disorder. In one embodiment, the pharmaceutical compositions provided herein are for use in the treatment of a muscle-related disorder selected from or caused by muscle atrophy, orthopedic injury, muscle injury, muscle weakness, heart failure, chemotherapy, and post-stroke, secondary muscle loss, sarcopenia, cachexia, obesity or metabolic syndrome.

In one embodiment the composition is formulated together with a pharmaceutically acceptable carrier, excipient or diluent.

Dosage regimens are adjusted to provide the optimum desired response (e.g. a therapeutic response). For example, a single bolus may be administered, several divided doses may be administered over time or the dose may be proportionally reduced or increased as indicated by the exigencies of the therapeutic situation. Dosage unit form as used herein refers to physically discrete units suited as unitary dosages for the subjects to be treated; each unit contains a predetermined quantity of active compound calculated to produce the desired therapeutic effect in association with the required pharmaceutical carrier. The specification for the dosage unit forms of the invention are dictated by and directly dependent on the unique characteristics of the active compound and the particular therapeutic effect to be achieved, and the limitations inherent in the art of compounding such an active compound for the treatment of sensitivity in individuals. A therapeutically effective amount of C1orf54 polypeptides or variants thereof or pharmaceutical composition comprising said C1orf54 polypeptides or variants thereof, ranges from about 0.001 to 150 mg/kg, or 0.01 to 30 mg/kg, and more usually 0.1 to 10 mg/kg of the host body weight. The skilled person knows to identify a suitable effective dose, which will vary depending on the route of administration (e.g. intravenously or subcutaneously). In some methods of systemic administration, dosage is adjusted to achieve a plasma concentration of C1orf54 polypeptides or variants thereof of 1-1000 µg/ml and in some methods 25-500 µg/ml. Alternatively, the composition can be a sustained release formulation, in which case less frequent administration is required. Dosage and frequency vary depending on the half-life of the C1orf54 polypeptide or variant thereof in the patient. The dosage and frequency of administration can vary depending on whether the treatment is prophylactic or therapeutic. In prophylactic applications, e.g. a relatively low dosage is administered at relatively infrequent intervals over a long period of time. In therapeutic applications, e.g. a relatively high dosage at relatively short intervals is sometimes required until progression of the disease is reduced or terminated or until the patient shows partial or complete amelioration of symptoms of disease. Thereafter, the patient can be administered a prophylactic regimen. Actual dosage levels of the active agents in the pharmaceutical compositions provided herein may be varied so as to obtain an amount of the active agent which is effective to achieve the desired therapeutic response for a particular patient, composition, and mode of administration, without being toxic to the patient. The selected dosage level will depend upon a variety of pharmacokinetic factors including the activity of the particular compositions of the present invention employed, or the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors known in the medical arts. An exemplary treatment regime entails administration at least once per day, at least once per week, at least once every two weeks, at least once every three weeks, at least once every four weeks, at least once a month, at least once every 3 months or at least once every three to 6 months. Administration of a therapeutically effective dose of C1orf54 polypeptides or variants thereof comprised in the compositions of the invention can result in a decrease in severity of disease symptoms, an increase in frequency and duration of disease symptom-free periods, or a prevention of impairment or disability due to the disease affliction, i.e. an increase muscle strength and/or regenerative capacity of the muscle.

Patients will receive an effective amount of the C1orf54 polypeptide or variant thereof, i.e. an amount that is sufficient to treat, ameliorate, or prevent the muscle-related disorder in question. Therapeutic effects may also include reduction in physical symptoms. Therapeutic effects may mimic effects of exercise. Dosage can be by a single dose schedule or a multiple dose schedule. Dosage can be intermittently. During intermittent dosing a therapeutically effective dose of C1orf54 polypeptide or variant thereof, e.g. a polypeptide comprising an amino acid sequence of SEQ ID NOs:2, 3, or 4, e.g. comprised in pharmaceutical compositions, as described herein is administered to a patient, thereby inducing biological activity of C1orf54 as described herein, followed by a period in which the C1 orf54 polypeptide or variant thereof, e.g. a polypeptide comprising an amino acid sequence of SEQ ID NOs:2, 3, or 4, e.g. comprised in pharmaceutical compositions, is not administered to said patient, such that C1orf54 biological activity decreases compared to the induced state. In one embodiment, an intermittent dosing schedule comprises at least two cycles, each cycle comprising (a) a dosing period during which a therapeutically effective amount of C1orf54 polypeptide or variant thereof, e.g. a polypeptide comprising an amino acid sequence of SEQ ID NOs:2, 3, or 4, e.g. comprised in pharmaceutical compositions, as described herein is administered to a patient and thereafter (b) a resting period.

A composition of the present invention can be administered by one or more routes of administration using one or more of a variety of methods known in the art. As will be appreciated by the skilled artisan, the route and/or mode of administration will vary depending upon the desired results. Routes of administration for the therapeutic proteins of the invention include intravenous, intramuscular, intradermal, intraperitoneal, subcutaneous, local delivery, spinal or other parenteral routes of administration, for example by injection or infusion. In one embodiment the C1orf54 polypeptides or variant thereof or composition comprising said C1orf54 polypeptide or variant thereof are administered intravenously. In another embodiment the C1orf54 polypeptides or variant thereof or composition comprising said C1orf54 polypeptide or variant thereof are administered subcutaneously. In another embodiment the C1orf54 polypeptides or variant thereof or composition comprising said C1orf54 polypeptide or variant thereof are administered intramuscularly.

### USES AND METHODS OF THE INVENTION

The C1orf54 polypeptides or variants thereof, e.g. as disclosed herein or compositions comprising said C1 orf54 polypeptides or variants thereof have therapeutic utilities. For example, these molecules can be administered to a subject and may be used in the treatment of a disease, prophylaxis and/or for delaying the onset of disease symptoms. The invention provides the C1orf54 polypeptides or variants thereof, e.g. as disclosed herein, or compositions comprising said C1 orf54 polypeptides or variants thereof for use in therapy or as a medicament. The invention further provides the C1orf54 polypeptides or variants thereof, e.g. as disclosed herein, or compositions comprising said C1 orf54 polypeptides or variants thereof for use in the treatment of a pathological disorder, e.g. a muscle-related disorder. The invention further provides use of the C1orf54 polypeptides or variants thereof, e.g. as disclosed herein, or compositions comprising said C1 orf54 polypeptides or variants thereof in the manufacture of a medicament for the treatment of a pathological disorder, e.g. a muscle-related disorder. In one embodiment, the invention relates to the C1 orf54 polypeptides or variants thereof, e.g. as disclosed herein, or compositions comprising said C1 orf54 polypeptides or variants thereof for use in increasing muscle function. In one particular embodiment, said muscle function comprises increased muscle strength. In a particular embodiment, the invention relates to the C1orf54 polypeptides or variants thereof, e.g. as disclosed herein, or compositions comprising said C1orf54 polypeptides or variants thereof for use in increasing muscle strength in a patient in need thereof. The need to increase muscle strength can result from one of the conditions mentioned herein, particularly as a consequence of a muscle-related disorder or it can result from a condition, e.g. a pathological condition, not mentioned herein, which nonetheless benefits from increased muscle function. In another embodiment, said muscle function comprises improved muscle regeneration. In another embodiment, said muscle function comprises increased muscle strength and improved muscle regeneration.

In one aspect, the invention provides C1orf54 polypeptides or variants thereof as described herein for use in therapy. Accordingly, the invention provides a polypeptide selected from:
a. a polypeptide consisting of amino acid sequence SEQ ID NO:2, 3, or 4;
b. a polypeptide comprising an amino acid sequence of SEQ ID NO:2, 3, or 4;
c. a polypeptide consisting of an amino acid sequence having at least 95% identity to SEQ ID NOs:2, 3, or 4;
d. a polypeptide comprising an amino acid sequence having at least 95% identity to SEQ ID NOs:2, 3, or 4; and
e. a polypeptide comprising or consisting of an amino acid sequence, wherein one, two, three, four or five amino acids have been modified, deleted, added and/or substituted within the sequence of any one of SEQ ID NOs:2, 3, or 4,
for use as a medicament.

In one embodiment, the C1orf54 polypeptides or variants thereof, e.g. a polypeptide comprising an amino acid sequence of SEQ ID NOs:2, 3, or 4, have myokine activity. In a further embodiment, the C1orf54 polypeptides or variants thereof, e.g. a polypeptide comprising an amino acid sequence of SEQ ID NOs:2, 3, or 4, are for use in the treatment of a muscle-related disorder in a subject.

In another aspect, the invention provides C1orf54 polypeptides or variants thereof as described herein for use in the manufacture of a medicament. The invention provides a polypeptide selected from:
a. a polypeptide consisting of amino acid sequence SEQ ID NO:2, 3, or 4;
b. a polypeptide comprising an amino acid sequence of SEQ ID NO:2, 3, or 4;
c. a polypeptide consisting of an amino acid sequence having at least 95% identity to SEQ ID NOs:2, 3, or 4;
d. a polypeptide comprising an amino acid sequence having at least 95% identity to SEQ ID NOs:2, 3, or 4; and
e. a polypeptide comprising or consisting of an amino acid sequence, wherein one, two, three, four or five amino acids have been modified, deleted, added and/or substituted within the sequence of any one of SEQ ID NOs:2, 3, or 4,
for use in the manufacture of a medicament.

In one embodiment, the C1orf54 polypeptides or variants thereof, e.g. a polypeptide comprising an amino acid sequence of SEQ ID NOs:2, 3, or 4, for use in the manufacture of a medicament have myokine activity. In a further embodiment, the C1orf54 polypeptides or variants thereof, e.g. a polypeptide comprising an amino acid sequence of SEQ ID NOs:2, 3, or 4, are for use in the manufacture of a medicament for the treatment of a muscle-related disorder in a subject.

In a further aspect, the invention provides a polypeptide or variant thereof for use in treating a muscle-related disorder in a subject, wherein the treatment comprises administering to the subject a polypeptide selected from:
a. a polypeptide consisting of amino acid sequence SEQ ID NO:2, 3, or 4;
b. a polypeptide comprising an amino acid sequence of SEQ ID NO:2, 3, or 4;
c. a polypeptide consisting of an amino acid sequence having at least 95% identity to SEQ ID NOs:2, 3, or 4;
d. a polypeptide comprising an amino acid sequence having at least 95% identity to SEQ ID NOs:2, 3, or 4; and
e. a polypeptide comprising or consisting of an amino acid sequence, wherein one, two, three, four or five amino acids have been modified, deleted, added and/or substituted within the sequence of any one of SEQ ID NOs:2, 3, or 4;
and wherein said polypeptide has myokine activity.

In another aspect, the invention provides a polypeptide or variant thereof for use in treating a muscle-related disorder in a subject, wherein the treatment comprises administering to the subject an effective amount of a polypeptide selected from:
f. a polypeptide consisting of amino acid sequence SEQ ID NO:2, 3, or 4;
g. a polypeptide comprising an amino acid sequence of SEQ ID NO:2, 3, or 4;
h. a polypeptide consisting of an amino acid sequence having at least 95% identity to SEQ ID NOs:2, 3, or 4; or
i. a polypeptide comprising an amino acid sequence having at least 95% identity to SEQ ID NOs:2, 3, or 4; wherein said polypeptide has myokine activity.

The invention further provides a method of treating a patient suffering from a pathological disorder, e.g. a muscle-related disorder, comprising administering a therapeutically effective amount of a C1orf54 polypeptide or variant thereof, e.g. a polypeptide comprising an amino acid sequence of SEQ ID NOs: 2, 3, or 4, or pharmaceutical composition comprising said C1orf54 polypeptide or variant thereof, e.g. a polypeptide comprising an amino acid sequence of SEQ ID NOs: 2, 3, or 4, to said patient. Accordingly, the present invention provides a method of treating a muscle-related disorder comprising administering a therapeutically effective amount of a C1orf54 polypeptide or variant thereof, e.g. a polypeptide with SEQ ID NO:2, 3 or 4, e.g. comprised in a pharmaceutical composition, having myokine activity as described herein, whereby said myokine activity comprises muscle function increasing activity. Said muscle function comprises muscle strength and/or muscle regeneration. In a particular embodiment, the invention relates to a method for increasing muscle strength. In another embodiment, said muscle function comprises improved muscle regeneration. In another embodiment, said muscle function comprises increased muscle strength and improved muscle regeneration. The need to increase muscle strength can result from one of the conditions mentioned herein, particularly as a consequence of a muscle-related disorder or it can result from a condition, e.g. a pathological condition, not mentioned herein, which nonetheless benefits from increased muscle function.

Accordingly, the invention provides a method of treating a muscle-related disorder comprising administering C1orf54 polypeptides or variants thereof as described herein. The invention provides a method of treating a muscle-related disorder in a subject, wherein said method comprises administering to the subject a therapeutically effective amount of a polypeptide selected from:
a. a polypeptide consisting of amino acid sequence SEQ ID NO:2, 3, or 4;
b. a polypeptide comprising an amino acid sequence of SEQ ID NO:2, 3, or 4;
c. a polypeptide consisting of an amino acid sequence having at least 95% identity to SEQ ID NOs:2, 3, or 4;
d. a polypeptide comprising an amino acid sequence having at least 95% identity to SEQ ID NOs:2, 3, or 4; and
e. a polypeptide comprising or consisting of an amino acid sequence, wherein one, two, three, four or five amino acids have been modified, deleted, added and/or substituted within the sequence of any one of SEQ ID NOs:2, 3, or 4;
and wherein said polypeptide has myokine activity.

In the following sections, various aspects and embodiments of the uses and methods stated above are described and all these aspects and embodiments can be combined together. The skilled person realizes that the teaching disclosed on the following pages are all combinable with each other and particular aspects and embodiments combining features from various parts of these pages will be considered to be adequately disclosed to the skilled person.

The isolated C1orf54 polypeptides or variants thereof, e.g. a polypeptide comprising an amino acid sequence of SEQ ID NOs: 2, 3, or 4, e.g. comprised in pharmaceutical compositions, of the present invention exhibit myokine activity, e.g. as indicated in vitro and *in vivo* tests as provided herein, e.g. as described by the modes for carrying out the invention, and are therefore indicated for therapy or for use *in vivo* or *in vitro.* Especially contemplated are diseases and disorders that benefit from an increase in muscle function such as muscle strength and/or from improved muscle regeneration.

Accordingly, the present invention provides C1orf54 polypeptides and variants thereof, e.g. a polypeptide comprising an amino acid sequence of SEQ ID NOs: 2, 3, or 4, e.g. comprised in pharmaceutical compositions, as described herein for use in increasing muscle strength.

The present invention also provides C1orf54 polypeptides and variants thereof, e.g. a polypeptide comprising an amino acid sequence of SEQ ID NOs: 2, 3, or 4, e.g. comprised in pharmaceutical compositions, as described herein for use as a medicament for increasing muscle strength.

In addition, the present invention provides a method for increasing muscle strength in a subject in need thereof comprising administering an effective amount of a C1orf54 polypeptide or variant thereof, e.g. a polypeptide comprising an amino acid sequence of SEQ ID NOs: 2, 3, or 4, e.g. comprised in pharmaceutical compositions, as described herein.

Skeletal muscle is a tissue with high plasticity and high responsiveness to exercise. Satellite cells (muscle precursor cells) are typically activated upon exercise and injuries and play a key role in the remodeling of muscle tissue. MyoD is a key transcriptional factor that determines the fate of satellite cells, mainly by driving satellite cells into differentiation. Muscle precursor cells lacking MyoD resume a more stem-cell like phenotype and after injection into injured muscle, engraft with significantly higher efficiency compared to wild-type cells. In addition, satellite cells lacking MyoD can be detected underneath the basal lamina of muscle fibers and show increased proliferation and survival after engraftment (Asakura et al. (2007) Proc Natl Acad Sci USA 104, 16552-16557*).* It is therefore desirable to decrease MyoD transiently. The C1orf54 polypeptides of the present invention decrease the levels of MyoD in vitro (example 5, figure 5) or *in vivo* (example 4, figure 4).

Accordingly, provided herein are C1 orf54 polypeptides or variants thereof, e.g. a polypeptide comprising an amino acid sequence of SEQ ID NOs:2, 3, or 4, e.g. comprised in pharmaceutical compositions, having myokine activity for use in increasing muscle function in a subject in need thereof, comprising administering to the subject such polypeptides or pharmaceutical compositions, wherein administration of said polypeptides or pharmaceutical compositions decreases the levels of MyoD thereby promoting an increase in muscle function, suitably an increase in muscle strength and/or improved muscle regeneration, in the subject.

In another embodiment, provided is a method of treating a muscle-related disorder comprising administering a therapeutically effective amount of a C1orf54 polypeptide or variant thereof, e.g. a polypeptide comprising an amino acid sequence of SEQ ID NOs:2, 3, or 4, wherein administration of said polypeptide decreases the levels of MyoD thereby promoting an increase in muscle function, suitably an increase in muscle strength and/or improved muscle regeneration, in the subject.

In one embodiment, a C1orf54 polypeptide or variant thereof, e.g. a polypeptide comprising an amino acid sequence of SEQ ID NOs:2, 3, or 4, e.g. comprised in pharmaceutical compositions, as described herein is for use in stimulating the proliferation of myoblasts and/or for increasing the frequency or number of satellite cells. Said use may be an *in vivo* or an in vitro use, preferably an *in vivo* use.

Accordingly, in one embodiment, provided herein is a C1 orf54 polypeptide or variant thereof, e.g. a polypeptide comprising an amino acid sequence of SEQ ID NOs:2, 3, or 4, e.g. comprised in pharmaceutical compositions, having myokine activity for use in increasing muscle function in a subject in need thereof, comprising administering to the subject said polypeptide or pharmaceutical composition, wherein administration of said polypeptide or pharmaceutical composition stimulates the proliferation of myoblasts, thereby promoting an increase in muscle function, suitably an increase in muscle strength and/or improved muscle regeneration, in the subject.

In a further embodiment, the present invention also provides a C1orf54 polypeptide or variant thereof, e.g. a polypeptide comprising an amino acid sequence of SEQ ID NOs:2, 3, or 4, e.g. comprised in pharmaceutical compositions, having myokine activity for use in increasing muscle function in a subject in need thereof, comprising administering to the subject said polypeptide or pharmaceutical composition, wherein administration of the polypeptide or pharmaceutical composition increases the frequency or number of the subject's skeletal muscle stem cells or satellite cells, thereby promoting an increase in muscle function, suitably an increase in muscle strength and/or improved muscle regeneration, in the subject.

In another embodiment, the invention provides a method of treating a muscle-related disorder comprising administering a therapeutically effective amount of a C1orf54 polypeptide or variant thereof, e.g. a polypeptide comprising an amino acid sequence of SEQ ID NOs:2, 3, or 4, wherein administration of said polypeptide stimulates the proliferation of myoblasts, thereby promoting an increase in muscle function, suitably an increase in muscle strength and/or improved muscle regeneration, in the subject.

In yet another embodiment, the invention provides a method of treating a muscle-related disorder comprising administering a therapeutically effective amount of a C1orf54 polypeptide or variant thereof, e.g. a polypeptide comprising an amino acid sequence of SEQ ID NOs:2, 3, or 4, wherein administration of said polypeptide increases the frequency or number of the subject's skeletal muscle stem cells or satellite cells, thereby promoting an increase in muscle function, suitably an increase in muscle strength and/or improved muscle regeneration, in the subject.

The present invention also provides in one embodiment an *in vitro* method for promoting satellite cells self-renewal and/or differentiation, comprising the step of administering an effective amount of an isolated C1orf54 polypeptide or variant thereof, e.g. a polypeptide comprising an amino acid sequence of SEQ ID NOs:2, 3, or 4, e.g. comprised in pharmaceutical compositions, to an isolated biological sample comprising satellite cells. In a further embodiment provided is a method for stimulating the proliferation and/or differentiation of satellite cells into myoblasts, comprising contacting said cells with an amount of a C1orf54 polypeptide or variant thereof, e.g. a polypeptide comprising an amino acid sequence of SEQ ID NOs:2, 3, or 4, e.g. comprised in pharmaceutical compositions, for a time and under conditions sufficient for said satellite cells to proliferate and/or differentiate into myoblasts. In one embodiment, the invention also provides the *in vitro* use of a C1orf54 polypeptide or variant thereof, e.g. a polypeptide comprising an amino acid sequence of SEQ ID NOs:2, 3, or 4, e.g. comprised in pharmaceutical compositions, for promoting myoblast growth. In one embodiment, provided is an *in vitro* method for promoting myoblast growth, comprising administering an effective amount of a C1orf54 polypeptide or variant thereof, e.g. a polypeptide comprising an amino acid sequence of SEQ ID NOs:2, 3, or 4, e.g. comprised in pharmaceutical compositions, as described herein to an isolated biological sample comprising myoblasts. Said biological sample can be human skeletal muscle-derived cells (hSkMDC).

In another embodiment, the invention provides an *in vitro* screening method for identifying an agent or combination of agents promoting satellite cells self-renewal and/or differentiation, comprising the steps of:
a) contacting isolated satellite cells with a candidate agent or combination of candidate agents;
b) assessing the cell phenotype of said cells;
c) comparing the cell phenotype in step b) to the phenotype of satellite cells in the absence of said agent or combination of agents, and/or to the phenotype of satellite cells contacted with an isolated C1orf54 polypeptide or variant thereof, e.g. a polypeptide comprising an amino acid sequence of SEQ ID NOs:2, 3, or 4, e.g. comprised in pharmaceutical compositions.

According to a preferred embodiment, the C1orf54 polypeptide or variant thereof, e.g. a polypeptide comprising an amino acid sequence of SEQ ID NOs:2, 3, or 4, is for use in a subject affected with a muscle-related disorder. In another preferred embodiment, the invention provides pharmaceutical compositions comprising a C1 orf54 polypeptide or variant thereof, e.g. a polypeptide comprising an amino acid sequence of SEQ ID NOs:2, 3, or 4, for use in a subject affected with a muscle-related disorder. In another preferred embodiment, provided is a method of treating a subject with a muscle-related disorder comprising administering a therapeutically effective dose of a C1orf54 polypeptide or variant thereof, e.g. a polypeptide comprising an amino acid sequence of SEQ ID NOs:2, 3, or 4, as described herein.

A large number of muscle-related disorders have been shown to lead to muscle dysfunction. These muscle-related disorders include, but are not limited to, one or more musculoskeletal disease or disorder, such as muscle atrophy from all causes, myopathy including inherited or recessive myopathies (e.g. muscular dystrophies) and drug-induced myopathy (e.g. resulting from treatment with glucocorticoids), muscle-wasting diseases (such as cachexia that may be the result from underlying illnesses such as acquired immunodeficiency diseases [AIDS], congestive heart failure, rheumatoid arthritis, cancer, chronic obstructive pulmonary disease [COPD], liver failure or liver cirrhosis, burns, sepsis), protracted disuse (e.g. resulting from paralysis, nerve injury, coma, bed rest, and ICU stay), orthopedic injury, muscle injury, weakness induced by surgery (such as joint replacement surgery), chemotherapy, post-stroke, rhabdomyolysis, age-related conditions of muscle atrophy or attenuation (such as sarcopenia that may be the result of aging), obesity, metabolic syndrome, diabetes, cardiovascular injury or disease, endocrine disorder, or immunological disorder.

In certain embodiments the invention provides compounds and pharmaceutical compositions in a method or for use in the treatment of muscle-related disorders including, but not limited to, muscle atrophy from any cause, myopathy, muscle-wasting diseases, protracted disuse, orthopedic injury, muscle injury, muscle weakness, chemotherapy, post-stroke, chronic heart failure, rhabdomyolysis, age-related conditions, e.g. sarcopenia, secondary muscle loss, obesity and/or metabolic syndrome. Such use or method comprises administering to a subject in need thereof an effective amount of a C1 orf54 polypeptide or variant thereof, e.g. a polypeptide comprising an amino acid sequence of SEQ ID NOs:2, 3, or 4, e.g. comprised in pharmaceutical compositions, having myokine activity.

In one embodiment, the muscle-related disorder is muscle wasting. Muscle wasting is a general term used to describe a condition marked by the involuntary loss of skeletal muscle tissue resulting from a variety of diseases, disorders, other conditions, or events. There are many causes of muscle wasting, including myopathy, drug-induced myopathy, denervation due to nerve trauma or a result of degenerative, metabolic, or inflammatory neuropathy and age-related condition of muscle atrophy or attenuation.

In one embodiment, the muscle atrophy results from myopathy, such as myotonia; a congential myopathy, including nemalene myopathy, multi/minicore myopathy and myotubular (centronuclear) myopathy, benign congenital hypotonia, central core disease (also known as central core myopathy); mitochondrial myopathy (e.g. Kearns-Sayre syndrome); familial periodic paralysis; inflammatory myopathy; metabolic myopathy, such as caused by a glycogen or lipid storage disease; dermatomyositisis; polymyositis; inclusion body myositis (e.g. sporadic inclusion body myositis and hereditary inclusion body myopathy); myositis ossificans; rhabdomyolysis and myoglobinurias. The myopathy may be caused by a muscular dystrophy syndrome, such as Duchenne muscular dystrophy, Becker muscular dystrophy, myotonic muscular dystrophy, fascioscapulohumeral muscular dystrophy, Emery-Dreifuss muscular dystrophy, oculopharyngeal muscular dystrophy, limb-girdle muscular dystrophy, congenital muscular dystrophy (e.g. Merosin-deficient congenital muscular dystrophy, Bethlem myopathy, Ullrich congenital muscular dystrophy, Fukuyama congenital muscular dystrophy), spinal muscular dystrophy, rigid spine muscular dystrophy, distal muscular dystrophy, sarcoglycanopathies or hereditary distal myopathy.

In one embodiment, the muscle atrophy is drug-induced myopathy, e.g. as a result of treatment with glucocorticoids such as cortisol, dexamethasone, betamethasone, prednisone, methylprednisolone, or prednisolone.

In one embodiment, muscle atrophy is the result of a denervation due to nerve trauma or a result of degenerative, metabolic, or inflammatory neuropathy, including neuromuscular disease, disorder or injury (e.g. a motor neuron disease such as amyotrophic lateral sclerosis, infantile spinal muscular atrophy, juvenile spinal muscular atrophy, autoimmune motor neuropathy with multifocal conductor block), Parkinson's disease, Huntington's disease, myasthenia gravis, centronuclear myopathy (e.g. X-linked myotubular myopathy), autoimmune neurodegenerative diseases (e.g. Guillain Barre syndrome, chronic inflammatory demyelinating polyneuropathy, Lambert-Eaton myasthenia syndrome), Creutzfeldt-Jakob disease, and paralysis due to stroke or spinal cord injury.

In one embodiment, the muscle atrophy is an age-related condition of muscle atrophy or attenuation that benefit from an increase in muscle function. Examples of muscle-related disorders that may be treated include those associated with aging/senescence including, but are not limited to, sarcopenia, skin atrophy, muscle wasting, disuse muscular atrophy, atherosclerosis, arteriosclerosis, pulmonary emphysema, osteoporosis, osteoarthritis, immunologic incompetence, high blood pressure, dementia, Huntington's disease, Alzheimer's disease, cataracts, age-related macular degeneration, cancer, stroke, frailty, impaired kidney function, and age-related hearing loss.

In one embodiment of the invention, the muscle-related disorder results from skeletal immobilization (e.g. due to trauma, bed rest, voluntary inactivity, involuntary inactivity, injury), nutritional insufficiency (e.g. fasting or starvation), cardiomyopathy, myocardial infarction, angina, cancer, HIV wasting syndrome, or an endocrine disorder such as a thyroid gland or adrenal gland or pituitary gland disorder.

In one embodiment, the muscle-related disorder is a metabolic disorder, including Type II Diabetes, diabetes-associated muscle atrophy, metabolic Syndrome/Syndrome X/insulin resistance syndrome, hyperglycemia, obesity and obesity-associated sarcopenia.

In one embodiment, the muscle-related disorder is an acute muscle wasting disease such as seen in severe and moderate burn injury, including adult and pediatric burn injury suffering from loss of lean body mass and/or muscle wasting or patients suffering from sepsis and intensive-care unit (ICU) acquired weakness.

In one embodiment, the muscle-related disorder is cachexia that may be the result from underlying illnesses such as acquired immunodeficiency diseases [AIDS], congestive heart failure, rheumatoid arthritis, cancer, chronic obstructive pulmonary disease [COPD]) cirrhosis, burns, sepsis, multiple sclerosis, tuberculosis, human immunodeficiency virus, malnutrition, Parkinson's disease, emphysema, heart failure, motor neuron disease, cystic fibrosis, dementia, sarcopenia, chronic obstructive pulmonary disease (COPD), kidney disease, and kidney failure.

In one embodiment, the muscle-related disorder is a fracture of a limb (i.e. leg or arm), skull, spine, pelvis or joint (i.e. knee or hip). Thus, in one embodiment, the muscle-related disorder is an injury or fracture to one or more of humerus, radius, ulnar, carpal bone, metacarpal bone, clavicle, scapula, femur, os coxae, patella, tibia, fibula, talus, calcaneus, tarsal bone, metatarsal bone, ischium or ileum. In another embodiment, the muscle-related disorder is surgery on one or more of the following joints: knee, hip, ankle, shoulder, elbow, wrist. Such surgery includes replacement of one or more of hip, knee, shoulder or other joint(s). In one embodiment, the present invention also provides a C1orf54 polypeptide or variant thereof, e.g. a polypeptide comprising an amino acid sequence of SEQ ID NOs:2, 3, or 4, e.g. comprised in pharmaceutical compositions, in ameliorating the muscle wasting effects of enforced inactivity, e.g. resulting from surgery, e.g. hip or knee replacement surgery, comprising administering to the subject a polypeptide or pharmaceutical composition of the invention, wherein said polypeptide or pharmaceutical composition has myokine activity. In one embodiment, a patient may be pre-treated with a C1 orf54 polypeptide or variant thereof, e.g. a polypeptide comprising an amino acid sequence of SEQ ID NOs:2, 3, or 4, e.g. comprised in pharmaceutical compositions, prior to an anticipated period of enforced rest/inactivity. Such a period may occur when a patient is admitted to hospital, for example for surgery. The inactivity may be localized, such as by casting of a broken limb or joint, or by administration of a paralytic agent, or after surgery to the hip or leg or after joint replacement.

Treatment according to the methods and uses described herein may result in reduced muscle wasting in a subject with muscle-related disorder or at risk of a muscle-related disorder. It may also result in delayed muscle wasting in a subject with muscle-related disorder or at risk of a muscle-related disorder. It may also result in suppression of muscle wasting in a subject with muscle-related disorder or at risk of a muscle-related disorder. It may also result in maintaining muscle function in a subject with muscle-related disorder or at risk of a muscle-related disorder. It may also enhance functional recovery in a subject with muscle-related disorder or at risk of a muscle-related disorder. In one embodiment, provided is a C1 orf54 polypeptide or variant thereof, e.g. a polypeptide comprising an amino acid sequence of SEQ ID NOs:2, 3, or 4, e.g. comprised in pharmaceutical compositions, for enhancement of functional recovery post-stroke. In one embodiment, provided is a C1 orf54 polypeptide or variant thereof, e.g. a polypeptide comprising an amino acid sequence of SEQ ID NOs:2, 3, or 4, e.g. comprised in pharmaceutical compositions, for decreasing the time until functional recovery has been achieved in a patient post-stroke. In another embodiment, provided herein is a C1 orf54 polypeptide or variant thereof, e.g. a polypeptide comprising an amino acid sequence of SEQ ID NOs:2, 3, or 4, e.g. comprised in pharmaceutical compositions, for enhancement of functional recovery in a subject with acute muscle insult and associated muscle atrophy due to hip fracture. In another embodiment, provided herein is a C1 orf54 polypeptide or variant thereof, e.g. a polypeptide comprising an amino acid sequence of SEQ ID Nos:2, 3, or 4, e.g. comprised in pharmaceutical compositions, for enhancement of functional recovery in a subject of greater than 70 years of age. In another embodiment, provided herein is a C1orf54 polypeptide or variant thereof, e.g. a polypeptide comprising an amino acid sequence of SEQ ID NOs:2, 3, or 4, e.g. comprised in pharmaceutical compositions, for enhancement of functional recovery in a subject with ICU myopathy. In another embodiment, provided herein is a C1orf54 polypeptide or variant thereof, e.g. a polypeptide comprising an amino acid sequence of SEQ ID NOs:2, 3, or 4, e.g. comprised in pharmaceutical compositions, for enhancement of functional recovery in a subject with peripheral nerve injury. In another embodiment, provided herein is a C1orf54 polypeptide or variant thereof, e.g. a polypeptide comprising an amino acid sequence of SEQ ID NOs:2, 3, or 4, e.g. comprised in pharmaceutical compositions, for enhancement of functional recovery in a subject with orthopedic injury with immobilization. In another embodiment, provided herein is a C1orf54 polypeptide or variant thereof, e.g. a polypeptide comprising an amino acid sequence of SEQ ID NOs:2, 3, or 4, e.g. comprised in pharmaceutical compositions, for enhancement of functional recovery in a subject with muscle weakness and/or dysfunction associated with COPD. In another embodiment, provided herein is a C1 orf54 polypeptide or variant thereof, e.g. a polypeptide comprising an amino acid sequence of SEQ ID NOs:2, 3, or 4, e.g. comprised in pharmaceutical compositions, for enhancement of functional recovery in a subject with muscle weakness and/or dysfunction associated with heart failure. In another embodiment, provided herein is a C1 orf54 polypeptide or variant thereof, e.g. a polypeptide comprising an amino acid sequence of SEQ ID NOs:2, 3, or 4, e.g. comprised in pharmaceutical compositions, for enhancement of functional recovery in a subject with muscle weakness and/or dysfunction associated with chemotherapy. In another embodiment, provided herein is a C1orf54 polypeptide or variant thereof, e.g. a polypeptide comprising an amino acid sequence of SEQ ID NOs:2, 3, or 4, e.g. comprised in pharmaceutical compositions, for enhancement of functional recovery in a subject with muscle weakness and/or dysfunction associated with acute kidney injury (AKI). In another embodiment, provided herein is a C1 orf54 polypeptide or variant thereof, e.g. a polypeptide comprising an amino acid sequence of SEQ ID NOs:2, 3, or 4, e.g. comprised in pharmaceutical compositions, for enhancement of functional recovery in a subject with muscle weakness and/or dysfunction associated with chronic kidney injury (CKI).

In another embodiment, the present invention also provides a C1orf54 polypeptide or variant thereof, e.g. a polypeptide comprising an amino acid sequence of SEQ ID NOs:2, 3, or 4, e.g. comprised in pharmaceutical compositions, for enhancing muscle function, e.g. increased muscle strength and/or improved muscle regeneration and/or an increase in muscle fatigability resistance in a healthy subject.

Other patients who can benefit from the proposed treatment include patients recovering from acute or critical illness requiring intensive care or prolonged hospitalization; young adults recovering from severe trauma, such as motor vehicle accidents, moderate or severe burns, combat injuries. Since loss of muscle is a common complication of most illnesses that are either severe or prolonged, it is anticipated that increase of muscle strength and/or improved muscle regeneration will speed the recovery and return to function of patients who experience muscle loss regardless of the root cause of this loss.

In one embodiment, provided herein is a C1 orf54 polypeptide or variant thereof, e.g. a polypeptide comprising an amino acid sequence of SEQ ID NOs:2, 3, or 4, e.g. comprised in pharmaceutical compositions, preventing temporary or permanent disability in a subject immediately after injury to prevent secondary muscle loss.

In one embodiment, treatment with the methods and uses in accordance with the present invention will improve the subject's physical performance. Physical performance may be assessed by a variety of tests, including but not limited to, Short Physical Performance Battery (SPPB), gait speed measurement, timed get-up-and-go test (TGUG), stair climb power test (SCPT), 400 m walk test and/or 6-minute walk test for distance (6MWD).

The 6-minute walk test for distance (6MWD) as mentioned herein refers to the standard physical exercise test performed in accordance with the current clinical practice, e.g. as defined in the current practical guidelines provided by medical societies, e.g. the American Thoracic Society, e.g. as described in ATS Statement: Guidelines for the Six-Minute Walk Test, Am J Respir Crit Care Med Vol 166. pp 111-117, 2002.

The Short Physical Performance Battery (SPPB) as mentioned herein refers to the standard physical exercise test performed to assess lower extremity physical performance status by measures of three separate tests (standing balance, time to walk 8 feet (2.44 meters), and time to rise from a chair and return to the seated position 5 times) (PMID: 8126356). These three separate tests are then combined into a summary performance score on a scale from 0 to 12 SPPB points.

The gait speed measurement as mentioned herein refers to the standard physical exercise test performed to assess the subject's ability to walk a defined distance of between 8 feet (2.44 meters) and 6 meter walking distance. In a preferred embodiment, gait speed is assessed over a walking distance of 4 meters. Walking requires strength, coordination and balance, thereby placing demands on different organ systems, including the cardiovascular, respiratory, nervous, and musculoskeletal systems.

The timed get-up-and-go test (TUG) as mentioned herein refers to a standard physical exercise test performed to assess a subject's mobility. It measures the time that a person takes to rise from a chair, walk three meters, turn around, walk back to the chair, and sit down again and requires both static and dynamic strength. The test is performed in accordance with the recommendations by medical societies, e.g. the American Geriatrics Society/British Geriatrics Society (2011) (J Am Geriatr Soc 59, 148-157).

The stair climb power test (SCPT) as mentioned herein refers to a functional test for assessing a subject's lower-extremity strength, power, and balance. A number of test variations have been developed for different patient populations, such as knee or hip replacement (arthroplasty). Test variations include the number of steps, the height of the individual step (e.g. 18 cm or 20 cm), the task requirement (ascent only or ascent/decent combined), or whether the test is timed over a pre-determined number of steps or the step count is recorded for a given period of time, e.g. 9-step ascend/descend, 5-step ascend/descend 4-step ascend/descend, 30-second test, 6-step fast and self-paced.

In one embodiment, treatment with the methods and uses in accordance with the present invention will improve the subject's muscle strength. Muscle strength may be assessed by dynamic (e.g. 30 second chair stand test, stair climb test, knee flexion/extension) or static (e.g. hand grip dynamometry, isometric knee extension/flexion) movements across single or multiple joints and respiratory and other muscles (e.g. pulmonary function tests). Other commonly used tests for assessing muscle strength include bicycle ergometry, rapid dynamic contractions on resistance training machines and maximal vertical jumps

The 30 second chair stand test (Jones et al. (1999) Res Q Exerc Sport 70, 113-119) as mentioned herein refers to a standard physical exercise test performed to assess a subject's lower extremity strength and endurance. It measures the number of times a subject rises from a sitting position, coming to a full standing position and sitting down again in 30 seconds. It requires both lower extremity strength as well as endurance.

The handgrip test as mentioned herein refers to a standard physical exercise test performed to assess a subject's grip strength. Grip strength can be assessed with a hand dynamometer, e.g. a Jamar hand dynamometer (Lafayette Instrument Company, USA).

The knee flexion/extension test as mentioned herein refers to a standard physical exercise test performed to assess a subject's strength of the quadriceps (knee extension) and/or hamstrings (knee flexion). Isometric, isotonic or isokinetic strength can be assessed.

### KITS

The disclosure also encompasses kits for treating muscle-related disorders. Such kits comprise an C1 orf54 polypeptide or variant thereof, e.g. C1orf54 polypeptide or variant thereof, e.g. a polypeptide comprising SEQ ID NO:2, 3 or 4, (e.g. in liquid or lyophilized form) or a pharmaceutical composition comprising the C1 orf54 polypeptide or variant thereof, e.g. a polypeptide comprising SEQ ID NO:2, 3 or 4 as described herein. Additionally, such kits may comprise means for administering the C1orf54 polypeptide or variant thereof, e.g. a polypeptide comprising SEQ ID NO:2, 3 or 4 (e.g. an autoinjector, a syringe and vial, a prefilled syringe, a prefilled pen) and instructions for use. These kits may contain additional therapeutic agents for treating a muscle related disorder, e.g. for delivery in combination with the enclosed C1orf54 polypeptide or variant thereof, e.g. a polypeptide comprising SEQ ID NO:2, 3 or 4. Such kits may also comprise instructions for administration of the C1orf54 polypeptide or variant thereof, e.g. a polypeptide comprising SEQ ID NO:2, 3 or 4 to treat the patient with muscle-related disorder. Such instructions may provide the dose, route of administration, and dosing regimen for use with the enclosed C1 orf54 polypeptide or variant thereof, e.g. a polypeptide comprising SEQ ID NO:2, 3 or 4.

The phrase "means for administering" is used to indicate any available implement for systemically administering a drug to a patient, including, but not limited to, a pre-filled syringe, a vial and syringe, an injection pen, an autoinjector, an IV drip and bag, a pump, etc. With such items, a patient may self-administer the drug (i.e., administer the drug without the assistance of a physician) or a medical practitioner may administer the drug. Disclosed herein are kits for use treating a patient having a muscle-related disorder, comprising an C1orf54 polypeptide or variant thereof, e.g. a polypeptide comprising SEQ ID NO:2, 3 or 4 and means for administering the C1 orf54 polypeptide or variant thereof, e.g. a polypeptide comprising SEQ ID NO:2, 3 or 4 to the patient.

Disclosed herein are kits comprising C1orf54 polypeptide or variant thereof, e.g. a polypeptide comprising SEQ ID NO:2, 3 or 4 or a pharmaceutical composition comprising C1orf54 polypeptide or variant thereof, e.g. a polypeptide comprising SEQ ID NO:2, 3 or 4, and a pharmaceutically acceptable carrier, excipient or diluent, as described herein, wherein the kit additionally comprises instructions for use and means for administering said C1orf54 polypeptide or variant thereof, e.g. a polypeptide comprising SEQ ID NO:2, 3 or 4 or said pharmaceutical composition comprising said C1 orf54 polypeptide or variant thereof, e.g. a polypeptide comprising SEQ ID NO:2, 3 or 4, to a subject in need thereof.

### SEQUENCES

**Table 1: Sequence table**

| **SEQ ID Number** | **Feature** | **Sequence** |
|---|---|---|
| ***human C1orf54 (hsC1orf54)*** | | |
| **SEQ ID NO: 1** | Amino acid with signal peptide (aa1-131) | MDVLFVAIFAVPLILGQEYEDEERLGEDEYYQVVYYYTVTPSYDDF SADFTIDYSIFESEDRLNRLDKDITEAIETTISLETARADHPKPVTVKP VTTEPSPDLNDAVSSLRSPIPLLLSCAFVQVGMYFM |
| **SEQ ID NO: 15** | DNA encoding C1orf54 aa1-131 | ATGGATGTCCTCTTTGTAGCCATCTTTGCTGTGCCACTTATCCT GGGACAAGAATATGAGGATGAAGAAAGACTGGGAGAGGATGAA TATTATCAGGTGGTCTATTATTATACAGTCACCCCCAGTTATGAT GACTTTAGTGCAGATTTCACCATTGATTACTCCATATTTGAGTCA GAGGACAGGCTGAACAGGTTGGATAAGGACATAACAGAAGCAA TAGAGACTACCATTAGTCTTGAAACAGCACGTGCAGACCATCCG AAGCCTGTAACTGTGAAACCAGTAACAACGGAACCTAGTCCAGA TCTGAACGATGCCGTGTCCAGTTTGCGAAGTCCTATTCCCCTCC TCCTGTCGTGTGCCTTTGTTCAGGTGGGGATGTATTTCATG |
| **SEQ ID NO: 2** | Amino acid without signal peptide (aa17-131) | QEYEDEERLGEDEYYQVVYYYTVTPSYDDFSADFTIDYSIFESEDR LNRLDKDITEAIETTISLETARADHPKPVTVKPVTTEPSPDLNDAVSS LRSPIPLLLSCAFVQVGMYFM |
| **SEQ ID NO: 16** | DNA encoding C1orf54 aa17-131 | CAAGAATATGAGGATGAAGAAAGACTGGGAGAGGATGAATATTA TCAGGTGGTCTATTATTATACAGTCACCCCCAGTTATGATGACTT TAGTGCAGATTTCACCATTGATTACTCCATATTTGAGTCAGAGG ACAGGCTGAACAGGTTGGATAAGGACATAACAGAAGCAATAGA GACTACCATTAGTCTTGAAACAGCACGTGCAGACCATCCGAAG CCTGTAACTGTGAAACCAGTAACAACGGAACCTAGTCCAGATCT GAACGATGCCGTGTCCAGTTTGCGAAGTCCTATTCCCCTCCTCC TGTCGTGTGCCTTTGTTCAGGTGGGGATGTATTTCATG |

| ***Human C1orf54 variants*** | | |
|---|---|---|
| **SEQ ID NO: 3** | Amino acid (aa17-116) | QEYEDEERLGEDEYYQVVYYYTVTPSYDDFSADFTIDYSIFESEDR LNRLDKDITEAIETTISLETARADHPKPVTVKPVTTEPSPDLNDAVSS LRSPIP |
| **SEQ ID NO: 17** | DNA encoding C1orf54 aa17-116 | CAAGAATATGAGGATGAAGAAAGACTGGGAGAGGATGAATATTA TCAGGTGGTCTATTATTATACAGTCACCCCCAGTTATGATGACTT TAGTGCAGATTTCACCATTGATTACTCCATATTTGAGTCAGAGG ACAGGCTGAACAGGTTGGATAAGGACATAACAGAAGCAATAGA GACTACCATTAGTCTTGAAACAGCACGTGCAGACCATCCGAAG CCTGTAACTGTGAAACCAGTAACAACGGAACCTAGTCCAGATCT GAACGATGCCGTGTCCAGTTTGCGAAGTCCTATTCCC |
| **SEQ ID NO: 4** | Amino acid (aa67-131) | DKDITEAIETTISLETARADHPKPVTVKPVTTEPSPDLNDAVSSLRS PIPLLLSCAFVQVGMYFM |
| **SEQ ID NO: 18** | DNA encoding C1orf54 aa67-131 | GATAAGGACATAACAGAAGCAATAGAGACTACCATTAGTCTTGA AACAGCACGTGCAGACCATCCGAAGCCTGTAACTGTGAAACCA GTAACAACGGAACCTAGTCCAGATCTGAACGATGCCGTGTCCA GTTTGCGAAGTCCTATTCCCCTCCTCCTGTCGTGTGCCTTTGTT CAGGTGGGGATGTATTTCATG |

| **Construct 3: MRGS-His6-ENLYFQS-G-hsC1orf54 (aa17-131)** | | |
|---|---|---|
| **SEQ ID NO: 19** | DNA | ATGCGAGGTTCACATCATCATCATCATCATGAAAATTTATATTTT CAATCAGGTCAAGAATATGAAGATGAAGAACGATTAGGTGAAGA TGAATATTATCAAGTAGTTTATTACTATACTGTAACACCAAGTTAT GACGATTTTAGTGCTGATTTCACAATTGATTATTCAATTTTTGAAT CAGAGGATCGATTAAACCGTTTAGATAAAGATATTACAGAGGCA ATTGAAACAACAATTAGTCTGGAAACAGCACGTGCTGATCATCC AAAGCCAGTAACAGTTAAGCCAGTTACAACAGAACCTAGTCCTG ACCTTAATGATGCAGTAAGCAGTCTTCGTTCACCAATCCCACTT CTACTGTCATGTGCATTTGTTCAAGTTGGTATGTATTTTATG |
| **SEQ ID NO: 10** | Amino acid expressed | MRGSHHHHHHENLYFQSGQEYEDEERLGEDEYYQVVYYYTVTPS YDDFSADFTIDYSIFESEDRLNRLDKDITEAIETTISLETARADHPKP VTVKPVTTEPSPDLNDAVSSLRSPIPLLLSCAFVQVGMYFM |
| **SEQ ID NO: 5** | Amino acid purified | SGQEYEDEERLGEDEYYQVVYYYTVTPSYDDFSADFTIDYSIFESE DRLNRLDKDITEAIETTISLETARADHPKPVTVKPVTTEPSPDLNDA VSSLRSPIPLLLSCAFVQVGMYFM |

| **Construct 4: MRGS-His6-ENLYFQS-G-hsC1orf54 (aa67-131)** | | |
|---|---|---|
| **SEQ ID NO: 21** | DNA | ATGCGAGGTTCACATCATCATCATCATCATGAAAATTTATATTTT CAATCAGGTGATAAAGATATTACAGAGGCAATTGAAACAACAAT TAGTCTGGAAACAGCACGTGCTGATCATCCAAAGCCAGTAACA GTTAAGCCAGTTACAACAGAACCTAGTCCTGACCTTAATGATGC AGTAAGCAGTCTTCGTTCACCAATCCCACTTCTACTGTCATGTG CATTTGTTCAAGTTGGTATGTATTTTATG |
| **SEQ ID NO: 12** | Amino acid expressed | MRGSHHHHHHENLYFQSGDKDITEAIETTISLETARADHPKPVTVK PVTTEPSPDLNDAVSSLRSPIPLLLSCAFVQVGMYFM |
| **SEQ ID NO: 7** | Amino acid purified | SGDKDITEAIETTISLETARADHPKPVTVKPVTTEPSPDLNDAVSSL RSPIPLLLSCAFVQVGMYFM |

| **Construct 5: MRGS-His6-ENLYFQS-G-hsC1orf54 (aa17-116)** | | |
|---|---|---|
| **SEQ ID NO: 22** | DNA | ATGCGAGGTTCACATCATCATCATCATCATGAAAATTTATATTTT CAATCAGGTCAAGAATATGAAGATGAAGAACGATTAGGTGAAGA TGAATATTATCAAGTAGTTTATTACTATACTGTAACACCAAGTTAT GACGATTTTAGTGCTGATTTCACAATTGATTATTCAATTTTTGAAT CAGAGGATCGATTAAACCGTTTAGATAAAGATATTACAGAGGCA ATTGAAACAACAATTAGTCTGGAAACAGCACGTGCTGATCATCC AAAGCCAGTAACAGTTAAGCCAGTTACAACAGAACCTAGTCCTG ACCTTAATGATGCAGTAAGCAGTCTTCGTTCACCAATCCCA |
| **SEQ ID NO: 13** | Amino acid expressed | MRGSHHHHHHENLYFQSGQEYEDEERLGEDEYYQVVYYYTVTPS YDDFSADFTIDYSIFESEDRLNRLDKDITEAIETTISLETARADHPKP VTVKPVTTEPSPDLNDAVSSLRSPIP |
| **SEQ ID NO: 8** | Amino acid purified | SGQEYEDEERLGEDEYYQVVYYYTVTPSYDDFSADFTIDYSIFESE DRLNRLDKDITEAIETTISLETARADHPKPVTVKPVTTEPSPDLNDA VSSLRSPIP |

| **Construct 6: MG-hsC1orf54 (aa17-131)-GSS-Avi-His6** | | |
|---|---|---|
| **SEQ ID NO: 23** | DNA | ATGGGTCAAGAATATGAAGATGAAGAACGATTAGGTGAAGATGA ATATTATCAAGTAGTTTATTACTATACTGTAACACCAAGTTATGA CGATTTTAGTGCTGATTTCACAATTGATTATTCAATTTTTGAATCA GAGGATCGATTAAACCGTTTAGATAAAGATATTACAGAGGCAAT TGAAACAACAATTAGTCTGGAAACAGCACGTGCTGATCATCCAA AGCCAGTAACAGTTAAGCCAGTTACAACAGAACCTAGTCCTGAC CTTAATGATGCAGTAAGCAGTCTTCGTTCACCAATCCCACTTCT ACTGTCATGTGCATTTGTTCAAGTTGGTATGTATTTTATGGGTAG TTCAGGTTTGAATGATATCTTTGAAGCTCAAAAAATTGAGTGGCA CGAGCATCATCATCATCATCAT |
| **SEQ ID NO: 14** | Amino acid expressed | MGQEYEDEERLGEDEYYQVVYYYTVTPSYDDFSADFTIDYSIFESE DRLNRLDKDITEAIETTISLETARADHPKPVTVKPVTTEPSPDLNDA VSSLRSPIPLLLSCAFVQVGMYFMGSSGLNDIFEAQKIEWHEHHHH HH |
| **SEQ ID NO: 9** | Amino acid purified | GQEYEDEERLGEDEYYQVVYYYTVTPSYDDFSADFTIDYSIFESED RLNRLDKDITEAIETTISLETARADHPKPVTVKPVTTEPSPDLNDAV |
| | | SSLRSPIPLLLSCAFVQVGMYFMGSSGLNDIFEAQKIEWHEHHHHH H |

| **Construct 7: MS-hsC1orf54 (aa17-131)** | | |
|---|---|---|
| **SEQ ID NO:20** | DNA | ATGAGTCAAGAATATGAAGATGAAGAACGTTTAGGTGAAGATGA ATATTATCAGGTTGTTTACTACTACACTGTAACTCCAAGCTATGA CGACTTCAGTGCTGACTTCACCATCGACTACAGCATTTTTGAGT CAGAAGATCGTTTAAACCGTCTTGACAAAGACATCACCGAAGCA ATCGAAACCACCATTTCCCTGGAAACAGCTCGTGCTGATCACCC GAAACCAGTAACCGTTAAACCGGTGACTACTGAACCGTCTCCG GACCTGAACGACGCTGTGTCTTCTTTGCGTTCTCCGATTCCTCT GCTGCTGTCCTGTGCATTCGTTCAGGTTGGCATGTACTTCATG |
| **SEQ ID NO: 11** | Amino acid expressed | MSQEYEDEERLGEDEYYQVVYYYTVTPSYDDFSADFTIDYSIFESE DRLNRLDKDITEAIETTISLETARADHPKPVTVKPVTTEPSPDLNDA VSSLRSPIPLLLSCAFVQVGMYFM |
| **SEQ ID NO: 6** | Amino acid purified | SQEYEDEERLGEDEYYQVVYYYTVTPSYDDFSADFTIDYSIFESED RLNRLDKDITEAIETTISLETARADHPKPVTVKPVTTEPSPDLNDAV SSLRSPIPLLLSCAFVQVGMYFM |

| ***mouse C10rf54 (mC1orf54)*** | | |
|---|---|---|
| **SEQ ID NO: 24** | Ammo acid | MDVLFIALLVAPLILGQEYDHEEQLEEGDYYQVAYYYYTVTPNYDD FSVNFTVDYSVFESEDRLNRLNKEVTTTEAVETTASSYSLHTELMD PQNPVTTKPVTTEPVTTEPVTTEPQSPNQNDAMSTLQSPVSCFLL WTLLQGGVHFM |

| **Construct 1: MRGS-His6-ENLYFQS-G-mC1orf54 (aa17-148)** | | |
|---|---|---|
| **SEQ ID NO: 29** | DNA | ATGCGTGGTAGCCATCATCATCATCACCATGAAAATCTGTATTTT CAGAGCGGCCAAGAATATGATCACGAAGAACAGCTGGAAGAGG GTGATTATTATCAGGTTGCCTATTATTATTATACCGTGACCCCGA ACTATGATGATTTTAGCGTGAATTTCACCGTGGACTATAGCGTTT TTGAAAGCGAAGATCGTCTGAATCGCCTGAATAAAGAAGTTACC ACCACCGAAGCAGTTGAAACCACCGCCAGCAGCTATAGCCTGC ATACCGAACTGATGGACCCGCAGAATCCGGTGACCACCAAACC GGTTACAACCGAACCTGTTACCACGGAACCGGTGACGACAGAA CCGCAGAGCCCGAATCAGAATGATGCAATGAGCACCCTGCAGA GTCCGGTTAGCTGTTTTCTGCTGTGGACACTGCTGCAAGGTGG TGTTCATTTTATG |
| **SEQ ID NO: 27** | Amino acid expressed | MRGSHHHHHHENLYFQSGQEYDHEEQLEEGDYYQVAYYYYTVTP NYDDFSVNFTVDYSVFESEDRLNRLNKEVTTTEAVETTASSYSLHT |
| | | ELMDPQNPVTTKPVTTEPVTTEPVTTEPQSPNQNDAMSTLQSPVS CFLLWTLLQGGVHFM |
| **SEQ ID NO: 25** | Amino acid purified | SGQEYDHEEQLEEGDYYQVAYYYYTVTPNYDDFSVNFTVDYSVFE SEDRLNRLNKEVTTTEAVETTASSYSLHTELMDPQNPVTTKPVTTE PVTTEPVTTEPQSPNQNDAMSTLQSPVSCFLLWTLLQGGVHFM |

| **Construct 2: MG-mC1orf54 (aa17-148)-GSS-Avi-His6** | | |
|---|---|---|
| **SEQ ID NO:30** | DNA | ATGGGTCAAGAATATGATCACGAAGAACAGCTGGAAGAGGGTG ATTATTATCAGGTTGCCTATTATTATTATACCGTGACCCCGAACT ATGATGATTTTAGCGTGAATTTCACCGTGGACTATAGCGTTTTTG AAAGCGAAGATCGTCTGAATCGCCTGAATAAAGAAGTTACCACC ACCGAAGCAGTTGAAACCACCGCCAGCAGCTATAGCCTGCATA CCGAACTGATGGACCCGCAGAATCCGGTGACCACCAAACCGGT TACAACCGAACCTGTTACCACGGAACCGGTGACGACAGAACCG CAGAGCCCGAATCAGAATGATGCAATGAGCACCCTGCAGAGTC CGGTTAGCTGTTTTCTGCTGTGGACACTGCTGCAAGGTGGTGTT CATTTTATGGGATCCTCTGGCCTGAACGACATCTTCGAAGCCCA GAAAATCGAGTGGCACGAGCATCATCATCATCATCAT |
| **SEQ ID NO: 28** | Amino acid expressed | MGQEYDHEEQLEEGDYYQVAYYYYTVTPNYDDFSVNFTVDYSVF ESEDRLNRLNKEVTTTEAVETTASSYSLHTELMDPQNPVTTKPVTT EPVTTEPVTTEPQSPNQNDAMSTLQSPVSCFLLWTLLQGGVHFM GSSGLNDIFEAQKIEWHEHHHHHH |
| **SEQ ID NO: 26** | Amino acid purified | GQEYDHEEQLEEGDYYQVAYYYYTVTPNYDDFSVNFTVDYSVFES EDRLNRLNKEVTTTEAVETTASSYSLHTELMDPQNPVTTKPVTTEP VTTEPVTTEPQSPNQNDAMSTLQSPVSCFLLWTLLQGGVHFMGS SGLNDIFEAQKIEWHEHHHHHH |

| ***Wild type equine C1orf54 homolog*** | | |
|---|---|---|
| **SEQ ID NO: 31** | amino acid | MNIFFVAILAVPLILGQEYEDEEVLEEDDYYQVVYYYTVTPNYDDFG VNFTVDYSLFESEDRLNRLDKEVMEPAETTISRETEHADHQKPVTL KPVTMEP |

| ***Wild type canine C1orf54 homolog*** | | |
|---|---|---|
| **SEQ ID NO: 32** | amino acid | MDVLFVAILAVPLILGQEYEDGEGLEEDDYYQVVYYYTVTPNYDDF GANFTVDYSMFESEDRLNRLDKEVREAAETTISHETEGADPQKPE TLKPMTVEPILFIHERHRERERQRQKQAPHKEPDAHKEPDAGLNP GSLDQGPSRRQTLNR |

| ***Wild type bovine C1orf54 homolog*** | | |
|---|---|---|
| **SEQ ID NO: 33** | amino acid | MDVLFVAILAVPLILGQEYEDGEELEEDEYYQVIYYYTITPSYDEFG VNFTVDYSMFESENTVNRLDEKVEASETTISYETDRADHQKPVIEK PVTMEPQSPDLNDAVSGLQSPGPLLLSWALIQGVMYFL |

### MODES FOR CARRYING OUT THE INVENTION

It will be understood that the invention has been described by way of example only and modifications may be made whilst remaining within the scope and spirit of the invention.

### EXAMPLES

### Example 1: C1orf54 expression constructs

### (1) Design of expression constructs

Human and mouse C1orf54 constructs for recombinant expression were designed based on information available in the public domain (Zerbino et al. (2018) Nucleic Acids Research, 46(D1):D754-D761*;* The UniProt Consortium (2017), Nucleic Acids Research, 45(D1):D158-D169).

Full-length mature mouse C1orf54 residues Q17 to M148 (UniProt ID Q8R2K8), omitting the putative N-terminal signal peptide (residues M1 to G16), was either fused to an N-terminal MRGSHHHHHHENLYFQSG tag (SEQ ID NO:34) containing a TEV cleavage site (construct 1, see table 1 for construct information), or in between a N-terminal MG and a C-terminal GSSGLNDIFEAQKIEWHEHHHHHH tag (SEQ ID NO: 35) (construct 2).

Full-length mature human C1orf54 residues Q17 to M131 (UniProt ID Q8WWF1, (SEQ ID NO: 2), omitting the putative N-terminal signal peptide (residues M1 to G16), was either fused to an N-terminal MRGSHHHHHHENLYFQSG tag (SEQ ID NO:34) containing a TEV cleavage site (construct 3), or in between an N-terminal MG and a C-terminal GSSGLNDIFEAQKIEWHEHHHHHH tag (SEQ ID NO: 35) (construct 6), or only to an N-terminal MS peptide.

For the design of constructs 4 and 5, human C1orf54 was aligned with homologous sequences from other species (Figure 1). Sequences were retrieved from www.uniprot.org (The UniProt Consortium (2017), Nucleic Acids Research, 45(D1):D158-D169) or www.ensembl.org (Zerbino et al. (2018) Nucleic Acids Research, 46(D1):D754-D761), respectively, on August 18, 2015 and aligned in Jalview (Waterhouse et al. (2009) Bioinformatics, 25(9):1189-1191) using the MUSCLE algorithm (Edgar (2004) Nucleic Acids Research, 32(5):1792-1797). Construct 4 comprises residues D67 to M131 (SEQ ID NO:4), lacking the highly conserved and negatively charged N-terminal region, whereas construct 5 comprises residues Q17 to P116 (SEQ ID NO:3), lacking the C-terminal hydrophobic region. Both, construct 4 and 5, were fused to an N-terminal MRGSHHHHHHENLYFQSG tag (SEQ ID NO:34) containing a TEV cleavage site.

### (2) Cloning of constructs

The DNA sequence of mouse C1Orf54 was purchased from GeneArt (ThermoFisher) including the optimization for expression in *E. coli.* For generation of MRGS-His6-TEV-G-mC1Orf54 (aa17-148) (construct 1), the insert was amplified by PCR from the GeneArt construct adding MRGS-His6-TEV-G at the N-terminus using forward primer: 5'-GAAGGAGATATACATATGCGTGGTAGCCATCATCATCATCACCATGAAAATCTGTAT TTTCAGAGCGGCCAAGAATATGATCACGAAGAAC-3' (SEQ ID NO:36) and
reverse primer:5'- GTGGTGGTGGTGCTCGAGTGCGGCCGCTTATCACATAAAATGAA CACCACCTTGC-3' (SEQ ID NO:37).

The PCR product was digested by Ndel/Notl and ligated into the pET26b(+) expression vector treated with the same restriction enzymes. For construct 2 the MG-mC1Orf54 (aa17-148) insert was amplified by PCR from the same template using forward primer:
5'-GTTTAACTTTAAGAAGGAGATATACATATGGGTCAAGAATATGATCACGAA-3' (SEQ ID NO:38) and reverse primer: 5'-
GGCTTCGAAGATGTCGTTCAGGCCAGAGGATCCCATAAAATGAACACCACC-3' (SEQ ID NO:39).

The DNA fragment was cloned into a pET26b(+) backbone already containing GSS-Avi-His6 (and treated with the appropriate restriction enzymes Ndel/BamHI) via the In-Fusion technology.

In case of human C1Orf54, the DNA sequence of the mature part was optimized for expression in *E. coli* using an in-house algorithm before purchasing the gene synthesis of construct 6 (MG-hsC1Orf (aa17-131)-GSS-Avi-His6) at GeneArt including the Ndel restriction site at the 5' end and 2 stop codons as well as the NotI restriction site at the 3' end. The DNA fragment was digested by Nde/Notl for subsequent ligation into the Ndel/Notl treated pET26b(+) expression vector. Constructs 3-5 and 7 were amplified from the previous construct 6 by PCR using the primers listed in Table 2. All 4 inserts were digested with Ndel/Notl and cloned in the same backbone used for the generation of construct #6.

**Table 2: PCR primers used to generate constructs 3-5 and 7 (for = forward primer, rev = reverse primer)**

| | |
|---|---|
| Construct 3 and 5 forward primer | GAAGGAGATATACATATGCGAGGTTCACATCATCATCATCATCATG AAAATTTATATTTTCAATCAGGTCAAGAATATGAAGATGAAG (SEQ ID NO:40) |
| Construct 3 and 4 reverse primer | GTGCTCGACGATATCGCGGCCGCTTATCACATAAAATACATACC (SEQ ID NO:41) |
| Construct 5 reverse primer | GTGGTGCTCGACGATATCGCGGCCGCTTATCATGGGATTGGTGAA CG (SEQ ID NO:42) |
| Construct 4 forward primer | CTTTAAGAAGGAGATATACATATGCGAGGTTCACATCATCATCATC ATCATGAAAATTTATATTTTCAATCAGGTGATAAAGATATTACAGAG GC (SEQ ID NO:43) |
| Construct 7 forward primer | CTTTAAGAAGGAGATATACATATGAGTCAAGAATATGAAG (SEQ ID NO:44) |
| Construct 7 reverse primer | GCTCGACGATATCGCGGCCGCTTATCACATGAAGTACATGCC (SEQ ID NO:45) |

In each case the ligation product was transfected into competent DH5α *E. coli* cells. Several colonies growing on LB/Kanamycin plates were selected and a plasmid DNA preparation was carried out. Positive clones were identified afterwards by sequence

### (3) Expression of constructs

Expression plasmids were transformed into *Escherichia coli* strain BL21-Gold (DE3) (Agilent). For all constructs, 1-liter Terrific Broth media was inoculated with an over-night pre-culture. Fermentation was performed in a DASGIP bioreactor system (Eppendorf). Cultures were grown at 37°C until the trigger point of pH 7.01 was reached. At this point, expression was induced by the addition of 1 mM IPTG. For constructs 1, 2, 4, and 7 cultivation continued at 37°C for 5 hours. For constructs 3, 5, and 6 temperature was lowered to 20°C and cultivation continued for 14 hours. At the end of the cultivation phase, cultures were harvested by centrifugation. Cell pellets were stored at -20°C and delivered to purification for further downstream processing.

### (4) Isolation of recombinant protein

Recombinant expression of constructs 1, 2, 3, 4, 6, and 7 in *Escherichia coli* lead to formation of inclusion bodies. *E. coli* cells containing either of these constructs were suspended in 0.1 M Tris/HCl, 0.1 M NaCl, 1 mM EDTA, 3 mM L-methionine, pH 7.0, using 10 ml buffer for 1 g of wet cell pellet, and supplemented with 1 cOmplete^{™} protease inhibitor cocktail tablet (Roche) per 100 ml. 0.1 mg/ml of chicken egg white lysozyme were added and the suspension gently stirred at 4°C. After 30 min, 10 mM MgCl₂ and 10 U/ml Benzonase^{®} (Merck) were added and the suspension stirred for another 30 min at 4°C. Cells were lyzed by high pressure (2 passages at 800 bar and ambient temperature) using an APV 2000 laboratory homogenizer (SPX Flow). ½ a volume of 60 mM EDTA, 1.5 M NaCl, 5 % (v/v) Triton X-100, pH 7.0 was added to the lysate and gently stirred for 60 min at 4°C. Inclusion bodies were sedimented by centrifugation for 30 min at 10'000× *g* and 4°C. The pellet was washed 1x with 1 volume of 50 mM Tris/HCl, 20 mM EDTA, 0.6 M NaCl, 3 mM L-methionine, pH 7.0, and 3x with 1 volume 50 mM Tris/HCl, 0.3 M NaCl, pH 7.0, at 4°C, and centrifuged for 30 min at 10'000× *g* and 4°C after each washing step. Inclusion body pellets were stored at -20°C before further processing. For purification, 1 g of IBs were solubilized in 20 ml 6 M GdmCl, 10 mM Tris, 100 mM monobasic phosphate, adjusted with NaOH to pH 8.0 (buffer A1), with 5 mM DTT, overnight at ambient temperature. The resulting denatured protein solution was clarified by centrifugation for 30 min at ≥10'000× *g* at ambient temperature and sterile-filtered afterwards. The solution was loaded on a 5 ml HisTrap excel^{™} IMAC column (GE Healthcare) equilibrated with buffer A1, incl. 5 mM DTT, at 7°C. The column was washed with 100ml 10 mM Tris/HCl, 30 % isopropanol, pH 8.0, followed by 100 ml buffer A1. Bound protein was eluted with 6 M GdmCl, 10 mM Tris, 100 mM monobasic phosphate, adjusted with HCl to pH 4.5 (buffer A2) in a single step. 1.8 ml fractions were collected and analyzed by SDS-PAGE. Fractions containing the protein of interest were pooled. For refolding of the denatured protein, the pool was then mixed in a 1:1 ratio with 2 M L-Arg-HCl, supplemented with 5 mM DTT and dialyzed overnight against PBS, pH 7.3, containing 1 mM DTT. The dialyzed sample was concentrated to the desired concentration using PEG 35,000 (Brooks et al. (1962) Trans, 2(4):216-264) and sterile-filtered. Constructs 2 and 6, containing a C-terminal tag, were polished either by gel filtration or, directly after the denaturing IMAC, by RP-HPLC as described for the enzymatically cleaved constructs below.

Construct 5 was expressed soluble in the cytosol of *E*. *coli* cells containing this construct were suspended in 50 mM monobasic phosphate, 500 mM NaCl, 20 mM imidazole, adjusted with NaOH to pH 8 (buffer A2), supplemented with 1 cOmplete^{™} protease inhibitor cocktail tablet (Roche) per 50 ml, 30 U/ml Benzonase^{®} (Merck), and 10 mM MgCl₂, using 10 ml buffer for 1 g of wet cell pellet. Cells were lyzed by high pressure (2 passages at 800 bar and ambient temperature) using an APV 2000 laboratory homogenizer (SPX Flow). The lysate was centrifuged for 30 min at 10'000× *g* and 4 °C. The supernatant was filtered (0.22 um) and loaded overnight on a 5 ml HisTrap excel^{™} IMAC column (GE Healthcare) equilibrated with buffer A2. After washing to A280 baseline signal, the protein was eluted by a liner gradient from 20 to 500 mM imidazole in buffer A2 over 10 column volumes. 1.8 ml fractions were collected and analyzed by SDS-PAGE. Fractions containing the protein of interest were pooled and dialyzed overnight against PBS, pH 7.3, 1 mM DTT for enzymatic cleavage of the tag.

For constructs 1, 3, 4, and 5, the N-terminal tag was enzymatically cleaved off by addition of AcTEV^{™} protease (Thermo Fisher Scientific) to the respective sample (200 U/mg) followed by incubation for 3 h at 30 °C. Enzymatic cleavage was confirmed by MS. To facilitate separation of cleaved from remaining uncleaved protein and protease, the sample was dialyzed against buffer A1 with 5 mM DTT. The dialyzed protein was filtered and loaded on a 5 mL HisTrap excel^{™} IMAC column (GE Healthcare) equilibrated with buffer A1, incl. 5 mM DTT, and the flow-through was collected. The fractions containing the cleaved protein were pooled and either dialyzed overnight against PBS, pH 7.3, 1 mM DTT, followed by concentration using PEG 35,000, or loaded on a POROS^{™} 20 R1 (Thermo Fisher Scientific), 95x15 mm (17 ml) or 250x20 mm (78 ml),RP-HPLC column equilibrated with 25 mM ammonium bicarbonate at 8 ml/min and 50°C, and, after washing to A280 baseline signal with equilibration buffer, eluted at 8 ml/min and 50°C with a linear gradient from 0 to 75 % ethanol in 20 column volumes or with a step gradient to 75 % ethanol. Fractions containing the protein of interest were pooled and dialyzed for ≥24 h against PBS, pH 7.3 at 4 degrees, and finally sterile-filtered.

Inclusion bodies of construct 7 were solubilized in 6 M urea, 20 mM Tris/HCl, 5 mM EDTA, pH 8 (buffer A3), incl. 0.2 M sodium sulfite. The solution was centrifuged for 30 min at 10,000x *g*, sterile-filtered, purified by RP-HPLC as described above, dialyzed against PBS, and sterile-filtered.

### Example 2: Muscle C1orf54 mRNA is increased by exercise in rodents

**Method:** Male C57BL/6 mice at the age of 7-8 weeks were purchased from Charles River Laboratories. Mice were acclimatized to the research facility for seven days. They were housed at 25°C with a 12:12 h light-dark cycle and were fed a standard laboratory diet containing 18.2% protein and 3.0% fat with an energy content of 15.8 MJ/kg (Nafag, product # 3890, Kliba, Basel, Switzerland). Food and water were provided *ad libitum.* Sedentary control animals were housed in groups of two. Animals subjected to exercise were singly housed and had continuous access to running wheels (Low-Profile Wireless Running Wheel for Mouse, Med Associates Inc, Product #: ENV-047) for seven days. On day 8, mice from the groups with access to running wheels were additionally subjected to a single bout of exercise on a treadmill (Panlab, Harvard Apparatus) for 25 minutes and were then euthanized with CO₂ at specific time points, i.e. immediately, 30, 60 or 180 minutes post-treadmill exercise. Animals from the sedentary control group were euthanized on the same day. Quadriceps muscles were rapidly dissected and snap-frozen in liquid nitrogen. Total RNA was extracted from quadriceps muscle using standard procedures (TRIzol reagent (Invitrogen) and the QlAcube technology (Qiagen)). All samples were treated with DNase and RNA quality and amount were measured with a Bioanalyzer (Bioanalyzer 2100 Agilent). Libraries were generated using a ribosome RNA depletion protocol. Sequencing was performed using Illumina HiSeq2500 stranded paired end sequencing. Reads were mapped using star onto the mouse reference genome version mm10 and counts were generated using feature counts using gene level parameters. Reported counts are FPKM (Fragment per Kb per million).

**Result:** We applied a concurrent exercise paradigm consisting of voluntary wheel running and forced treadmill running to discover novel exercise-induced factors that are predicted to be secreted and identified C1orf54 as a so far uncharacterized factor: *C1orf54* mRNA levels increased in quadriceps muscles of exercised compared to sedentary mice. Indeed, elevated levels of *C1orf54* mRNA are detected in quadriceps muscles immediately after exercise and persist for at least 3 hrs (Figure 2), indicating that exercise stimulates the production of *C1orf54* mRNA in muscle.

### Example 3: Muscle C1ORF54 mRNA is increased in humans following exercise

**Method:** A publically available dataset (GSE97084) with transcriptomics data from *vastus lateralis* biopsies from humans undergoing various types of exercise (Robinson et al. (2017) Cell Metab 25, 581-592) was re-analyzed for expression levels of *C1ORF54.* Reported counts are FPKM (Fragment per Kb per million).

**Result:** Given the effect of exercise on *C1orf54* mRNA expression in mice, publically available datasets were analyzed to evaluate whether exercise regulates *C1ORF54* in a similar manner in humans. In a human exercise study where participants underwent different exercise paradigms (Robinson et al. (2017) Cell Metab 25, 581-592) *C1ORF54* mRNA levels in *vastus lateralis* were analyzed prior and post exercise for each study young participant. The data show that exercise increased the levels of *C1ORF54* in human skeletal muscle (Figure 3). The increase in *C1ORF54* mRNA is independent of the type of exercise regimen (high-intensity aerobic interval training (HIIT), resistance training (RT), and combined (CT) exercise training as described by (Robinson et al. (2017) Cell Metab 25, 581-592) and therefore reflects a general response to exercise.

### Example 4: C1orf54 regulates the transcription of Myod1 in vivo

**Method:** Male C57BL/6 mice at the age of 7-8 weeks were purchased from Charles River Laboratories. Mice were acclimatized to the research facility for seven days. They were housed at 25°C with a 12:12 h light-dark cycle and were fed a standard laboratory diet containing 18.2% protein and 3.0% fat with an energy content of 15.8 MJ/kg (Nafag, product # 3890, Kliba, Basel, Switzerland). Food and water were provided *ad libitum.* Mice received a single intraperitoneal injection of vehicle (PBS) or isolated polypeptide with amino acid sequence of SEQ ID NO:25 formulated in vehicle. 60 minutes after the injection, mice were euthanized with CO₂ and gastrocnemius muscles were excised and snap-frozen in liquid nitrogen. Total RNA was extracted from gastrocnemius muscles using TRIzol reagent (Invitrogen) according to manufacturer's instructions. Reverse transcription was performed with random hexamers on 1 µg of total RNA using a high-capacity reverse transcription kit (Applied Biosystems). Real-time PCR was performed in duplicates in 384-well plates on a C1000 Thermal cycler (CFX384 Real-Time system, Bio-Rad) using specific TaqMan probes for myogenic differentiation 1 (*Myod1,* Mm00440387_m1, Applied Biosystems) and two housekeeping genes (*Tbp,* Mm00446971_m1, Applied Biosystems and (*Hprt,* Mm03024075_m2, Applied Biosystems). Automatic settings were applied to determine the CT. The comparative method using 2^{-ΔΔCT} was used to determine the relative expression. Two housekeeping genes were used for normalization (*Tbp,* Mm00446971_m1, Applied Biosystems and *Hprt,* Mm03024075_m2, Applied Biosystems). All results are expressed as fold changes over vehicle.

Statistical analysis was carried out using one-way ANOVA followed by Fisher's least significant difference (LSD). Differences were considered to be significant when the probability value was < 0.05. Statistical analyses were performed by GraphPad Prism version 7.03 (GraphPad Software, Inc., La Jolla, CA).

**Results:** C1orf54 reduces the mRNA levels of *Myod1* in gastrocnemius 60 minutes after a single intraperitoneal injection of 0.5 or 1 mg/kg of isolated polypeptide with amino acid sequence of SEQ ID NO:25 as compared to vehicle (Figure 4). MyoD1 is a key transcriptional factor that determines the fate of satellite cells, mainly by driving satellite cells into differentiation. Satellite cells are involved in muscle growth, maintenance, repair and regeneration (Wang and Rudnicki (2011) Nat Rev Mol Cell Biol 13, 127-133). Muscle precursor cells lacking MyoD1 resume a more stem-cell like phenotype and after injection into injured muscle, engraft with significantly higher efficiency and show increased survival compared to wild-type cells. In addition, MyoD1 negative myoblast-derived satellite cells can be detected underneath the basal lamina of muscle fibers after engraftment, indicating self-renewal properties of MyoD1 negative myoblasts (Asakura et al. (2007) Proc Natl Acad Sci USA 104, 16552-16557*).* The activity of C1orf54, therefore, affects myogenic differentiation by downregulating MyoD1, thereby promoting a stem-cell like phenotype. C1orf54 therefore comprises muscle function improving activity.

### Example 5: C1orf54 inhibits the transcription of MYOD1 in human muscle cells in vitro

**Method:** Primary human muscle cells (hSkMDC, lot P401061-51M, Cook MyoSite, Pittsburgh, PA, USA) were thawed and seeded in two T175 tissue culture flasks in growth medium (GM) containing MyoTonic serum-free growth medium (COOK MK-2222), growth supplements (COOK MK-2288), 20% fetal bovine serum (Gibco, 16000-044, lot 1233705), 50 µg/mL gentamycin (Gibco, 15710-049) and 10 µg/mL insulin (Amimed, 5-79F00-G, lot K11988P). After 5 days of growth at 37°C/5% CO₂, cells were rinsed with DPBS (Gibco 14190-144), detached with Accutase (Gibco A11105-01) and counted using a nucleocounter (chemometec). 40'000 cells in 0.2 mL GM were seeded per well into 24-well plates coated with 0.5 mL gelatin (EmbryoMax 0.1% gelatin solution, Millipore ES-006-B) per well for 30 min at room temperature (RT). The next day, cells were treated with 125, 250, 500, 1000 ng/mL recombinant tag-free human C1 orf54 protein aa 17-131 (SEQ ID NO:2; Mybiosource, MBS 1284901 lot 03095), solvent control (33 µM Tris-HCl, 3.3 µM EDTA and 0.07% glycerol) or were not treated for 6h at 37°C. Subsequently, cells were lysed and total RNA extracted using Direct-zol kit according to manufacturer's instructions (Zymo research). cDNA was synthesized using High-Capacity cDNA archive kit (Applied Biosystems 4368813) and *MYOD1* gene expression was determined by quantitative PCR using TaqMan gene expression master mix (Applied Biosystems #4369016), TaqMan assay (Hs00159528_m1, Applied Biosystems) and a ABI PRISM 7000 sequence detection system (Applied Biosystems). The comparative method using 2^{-ΔΔCT} was used to determine the relative expression. Gene expression was normalized to the three housekeeping genes *GAPDH* (4310884E, Applied Biosystems), *TBP* (Hs00427620_m1, Applied Biosystems) and *HPRT* (Hs01003268_g1, Applied Biosystems).

**Result:** Recombinant human C1orf54 protein (aa17-131, SEQ ID NO:2) inhibited *MYOD1* gene expression in primary human muscle precursor cells in a dose-dependent manner after 6h treatment (Figure 5). The highest used dose (1000 ng/mL) yielded a 65.6% reduction in *MYOD1* gene expression (Figure 5). Myogenin, a late myogenic transcription factor, was not significantly regulated by C1 orf54 protein (data not shown). Muscle cells lacking MyoD1 show increased proliferation (Sabourin et al. (1999) J Cell Biol 144, 631-643). These data therefore indicate that C1 orf54 prevents myogenic differentiation, while promoting proliferation of muscle precursor cells.

### Example 6: C1orf54 increases muscle force in vivo

**Method:** Male C57BL/6 mice at the age of 12 weeks were purchased from Charles River Laboratories. Mice were acclimatized to the research facility for seven days, housed at 25°C with a 12:12 h light-dark cycle and fed a standard laboratory diet containing 18.2% protein and 3.0% fat with an energy content of 15.8 MJ/kg (Nafag, product # 3890, Kliba, Basel, Switzerland). Food and water were provided *ad libitum.*

Following acclimatization, mice were randomized by body weight and treated by intraperitoneal injections of vehicle (PBS) or 1 mg/kg of isolated polypeptide with amino acid sequence of SEQ ID NO:25 formulated in vehicle every third day. Each mouse received three injections in total (on day 1, 4 and 7, where day 1 is defined as the first day an animal receives an injection). On day 9 evoked muscle force was assessed noninvasively. To this end, the animals were anesthetized, the leg shaved and muscle contraction was evoked via electrical stimulation of the hind leg through transcutaneous electrodes. The electrodes were affixed to the quadriceps and the foot. Force was recorded on a homemade pedal connected to a force transducer (KD45.5N) and allowing recording the pressure of the foot upon stimulation. Frequencies ranging from 10 to 120 Hz were sequentially applied via the stimulator (Stimulator ML180, ADInstruments) and tetanic forces recorded at each frequency (PowerLab, ADInstruments).

Statistical analysis was carried out using multiple unpaired t-test comparison. Differences were considered to be significant when the probability value was <0.05. All statistical analyses were performed by GraphPad Prism version 7.03 (GraphPad Software, Inc., La Jolla, CA).

### Result:

At low stimulation frequencies ranging from 10-70 Hz (submaximal force generation), evoked force of the hind limb is similar between animals treated with vehicle and 1 mg/kg C1 orf54 (isolated polypeptide with amino acid sequence of SEQ ID NO:25) (Figure 6A). In contrast, at higher stimulation frequencies ranging from 80-120 Hz and reflecting maximal force generation, mice treated with C1orf54 display approximately 15% higher absolute muscle force compared to vehicle treated animals.

Specific force is assessed by normalizing absolute muscle force for the weight of the gastrocnemius muscle, which mainly contributes to the contraction monitored. Specific evoked force at higher stimulation frequencies is approximately 13% higher in C1 orf54 compared to vehicle treated animals (Figure 6B). This demonstrates that intermittent administration of C1orf54 polypeptide increases muscle strength.

### Example 7: C1orf54 increases the number of Pax7 positive satellite cells in vivo

**Method:** Male C57BL/6 mice at the age of 12 weeks were purchased from Charles River Laboratories. Mice were acclimatized to the research facility for seven days, housed at 25°C with a 12:12 h light-dark cycle and fed a standard laboratory diet containing 18.2% protein and 3.0% fat with an energy content of 15.8 MJ/kg (Nafag, product # 3890, Kliba, Basel, Switzerland). Food and water were provided *ad libitum.*

Following acclimatization, mice were randomized by body weight and treated with intraperitoneal injections of vehicle (PBS) or 1 mg/kg of isolated polypeptide with amino acid sequence of SEQ ID NO: 25 formulated in vehicle every third day. Each mouse received four injections in total (on day 1, 4, 7 and 10, where day 1 is defined as the first day an animal receives an injection). On day 11, animals were euthanized with CO₂ and the gastrocnemius muscle was rapidly dissected out, embedded in OCT (Tissue-Tek) and frozen in liquid nitrogen-cooled isopentane.

Frozen muscles were cut into 8 µm cross-sections, which were dried at room temperature for ~2 hour and stored at -20°C in a plastic box tightly sealed in a bag. On the day of the staining, slides were taken out of the freezer, dried at room temperature for 30 minutes and fixed with 3% paraformaldehyde for 10 min. Sections were then blocked with Vector M.O.M blocking reagent (Vector Laboratories, Cat. #MKB-2213, diluted 1/100) and 10% goat serum in PBTX (PBS containing 0.2% Triton X100) for 30 minutes. To detect satellite cells, the sections were incubated overnight with a primary antibody against Pax7 (Developmental Studies Hybridoma Bank (DSHB)) in blocking buffer at 4°C. The Pax7 hybridoma developed by A. Kawakami at the Tokyo Institute of Technology was obtained from the DSHB, created by the NICHD of the NIH and maintained at The University of Iowa, Department of Biology, Iowa City, IA 52242. The mouse antibody was purified at Novartis from hybridoma and detects the antigen amino acids 352-523 of chicken Pax7). After several washes with PBTX, the sections were incubated at room temperature with a bridging antibody (rat anti-mouse IgG, Serotec, Cat. #MCA336, diluted 1/500) in 10% goat serum in PBTX for 3 hours. Samples were subsequently washed with PBTX and stained with a fluorescently labeled secondary antibody (Alexa 594 goat anti-rat, Invitrogen, Cat. #A11007, diluted 1/200) at room temperature for 1 hour. After washing with PBTX, samples were mounted with ProLong Gold antifade reagent with DAPI (Invitrogen). Cells positive for both Pax7 and DAPI were counted. Statistical analysis was carried out using unpaired t-test. Differences were considered to be significant when the probability value was <0.05. Statistical analyses was performed by GraphPad Prism version 7.03 (GraphPad Software, Inc., La Jolla, CA).

**Result:** To determine the number of satellite cells, muscle sections were stained for Pax7, which represents a well-established marker for satellite cells (Kuang and Rudnicki (2008) Trends Mol Med 14, 82-91*).* Satellite cells are involved in muscle growth, maintenance, repair and regeneration (Wang and Rudnicki (2011) Nat Rev Mol Cell Biol 13, 127-133). Intermittent treatment with 1 mg/kg C1orf54 on day 1, 4, 7 and 10 significantly increased the number of Pax7 positive satellite cells in gastrocnemius by approximately 66% compared to vehicle treatment (Figure 7). The increased number of satellite cells following treatment with C1orf54 demonstrates that C1orf54 promotes muscle growth and regenerative capacity of skeletal muscle.

### Example 8: C1orf54 promotes proliferation of human myoblasts in vitro

**Method:** Primary human muscle cells (hSkMDC, lot P201076-19M) from Cook MyoSite, Pittsburgh, PA, USA were thawed and seeded in three T175 tissue culture flasks in growth medium (GM) containing MyoTonic serum-free growth medium (COOK MK-2222), growth supplements (COOK MK-8888), 10% fetal bovine serum superior (Millipore, S0613, lot 15H095) and 50 µg/mL gentamycin (Gibco, 15710-049). After two days of growth at 37°C/5% CO₂, cells were rinsed with DPBS (Gibco 14190-144), detached with Accutase (A11105-01) and counted using a nucleocounter (ChemoMetec). 1 '000 cells in 0.1 mL GM were seeded per well into 96-well plates coated with 0.1 mL gelatin (EmbryoMax 0.1% gelatin solution, Millipore ES-006-B) per well for 30 min at RT. After overnight incubation, cells were incubated with GM without FBS for 2.5 h, followed by medium change into GM containing 1% FBS for 2.5 h. Subsequently, cells were treated with 1000 ng/mL recombinant tag-free human C1orf54 protein aa 17-131 (SEQ ID NO:2; Mybiosource, MBS 1284901 lot 03095), C1orf54 MBS solvent control (53 µM Tris-HCl, 5.3 µM EDTA and 0.26% glycerol) or 1% PBS control. Cell growth as percent (%) confluence was measured by life cell imaging (2 images per well every 4h) using an IncuCyte FL Live-Cell analysis system (Essen BioScience, Ltd.).

Statistical analysis was carried out using multiple unpaired t-test comparison. All statistical analyses were performed by GraphPad Prism version 7.03 (GraphPad Software, Inc., La Jolla, CA).

**Result:** Recombinant human C1orf54 protein (SEQ ID NO:2; aa17-131) enhanced the growth of human myoblasts clearly and persistently starting 2 days after treatment until the end of the experiment at day 6 compared to control cells treated with C1orf54 protein solvent (Figure 8). Cells continuously grew under all conditions, but with different speeds. These data demonstrate that C1orf54 stimulates the growth of muscle precursor cells.

## Claims

1. A C1orf54 polypeptide for use in treating a muscle-related disorder in a subject, wherein the treatment comprises administering to the subject an effective amount of a polypeptide selected from:
a. a polypeptide consisting of amino acid sequence SEQ ID NO: 2, 3, or 4;
b. a polypeptide comprising an amino acid sequence of SEQ ID NO: 2, 3, or 4;
c. a polypeptide consisting of an amino acid sequence having at least 95% amino acid sequence identity to SEQ ID NOs: 2, 3, or 4;
d. a polypeptide comprising an amino acid sequence having at least 95% amino acid sequence identity to SEQ ID NOs: 2, 3, or 4; and
wherein said C1 orf54 polypeptide has myokine activity and wherein said muscle-related disorder is a disorder selected from or caused by muscle atrophy, orthopedic injury, muscle injury, muscle weakness, heart failure, chemotherapy, post-stroke, secondary muscle loss, sarcopenia, obesity or metabolic syndrome.

2. The C1 orf54 polypeptide for use according to claim 1, wherein the treatment comprises administering to the subject an effective amount of a polypeptide selected from:
a. a polypeptide consisting of amino acid sequence SEQ ID NO: 2;
b. a polypeptide comprising an amino acid sequence of SEQ ID NO: 2;
c. a polypeptide consisting of an amino acid sequence having at least 95% amino acid sequence identity to SEQ ID NOs: 2;
d. a polypeptide comprising an amino acid sequence having at least 95% amino acid sequence identity to SEQ ID NOs: 2.

3. The C1orf54 polypeptide for use according to claim 1 or 2, wherein said myokine activity comprises muscle function increasing activity.

## Patentansprüche

1. C1orf54-Polypeptid zur Verwendung beim Behandeln einer muskulären Erkrankung bei einem Individuum, wobei die Behandlung Verabreichen einer wirksamen Menge eines Polypeptids umfasst, das ausgewählt ist aus:
a. einem Polypeptid, das aus Aminosäuresequenz SEQ ID NO: 2, 3 oder 4 besteht;
b. einem Polypeptid, das eine Aminosäuresequenz unter SEQ ID NO: 2, 3 oder 4 umfasst;
c. einem Polypeptid, das aus einer Aminosäuresequenz besteht, die eine Aminosäuresequenzidentität von wenigstens 95 % mit SEQ ID NO: 2, 3 oder 4 aufweist;
d. einem Polypeptid, das eine Aminosäuresequenz umfasst, die eine Aminosäuresequenzidentität von wenigstens 95 % mit SEQ ID NO: 2, 3 oder 4 aufweist; und wobei das C1orf54-Polypeptid Myokinaktivität aufweist und wobei es sich bei der muskulären Erkrankung um eine aus Muskelatrophie, orthopädischer Verletzung, Muskelverletzung, Muskelschwäche, Herzinsuffizienz, Chemotherapie, Schlaganfallfolgen, sekundärem Muskelschwund, Sarkopenie, Fettleibigkeit oder metabolischem Syndrom ausgewählte oder dadurch verursachte Erkrankung handelt.

2. C1orf54-Polypeptid zur Verwendung nach Anspruch 1, wobei die Behandlung Verabreichen einer wirksamen Menge eines Polypeptids umfasst, das ausgewählt ist aus:
a. einem Polypeptid, das aus Aminosäuresequenz SEQ ID NO: 2 besteht;
b. einem Polypeptid, das eine Aminosäuresequenz unter SEQ ID NO: 2 umfasst;
c. einem Polypeptid, das aus einer Aminosäuresequenz besteht, die eine Aminosäuresequenzidentität von wenigstens 95 % mit SEQ ID NO: 2 aufweist;
d. einem Polypeptid, das eine Aminosäuresequenz umfasst, die eine Aminosäuresequenzidentität von wenigstens 95 % mit SEQ ID NO: 2 aufweist.

3. C1orf54-Polypeptid zur Verwendung nach Anspruch 1 oder 2, wobei die Myokinaktivität Muskelfunktion steigernde Aktivität umfasst.

## Revendications

1. Polypeptide Clorf54 pour une utilisation dans le traitement d'un trouble lié à un muscle chez un sujet, le traitement comprenant une administration au sujet d'une quantité efficace d'un polypeptide choisi parmi :
a. un polypeptide constitué d'une séquence d'acides aminés SEQ ID NO: 2, 3 ou 4 ;
b. un polypeptide comprenant une séquence d'acides aminés SEQ ID NO: 2, 3 ou 4 ;
c. un polypeptide constitué d'une séquence d'acides aminés ayant au moins 95 % d'identité de séquence d'acides aminés avec les SEQ ID NO: 2, 3 ou 4 ;
d. un polypeptide comprenant une séquence d'acides aminés ayant au moins 95 % d'identité de séquence d'acides aminés avec les SEQ ID NO: 2, 3 ou 4 ; et
ledit polypeptide Clorf54 ayant une activité de myokine et ledit trouble lié à un muscle étant un trouble choisi parmi, ou causé par, une atrophie musculaire, une lésion orthopédique, une lésion musculaire, une faiblesse musculaire, une défaillance cardiaque, une chimiothérapie, un post-AVC, une perte de muscle secondaire, une sarcopénie, l'obésité ou un syndrome métabolique.

2. Polypeptide Clorf54 pour une utilisation selon la revendication 1, le traitement comprenant une administration au sujet d'une quantité efficace d'un polypeptide choisi parmi :
a. un polypeptide constitué d'une séquence d'acides aminés SEQ ID NO: 2 ;
b. un polypeptide comprenant une séquence d'acides aminés de SEQ ID NO: 2 ;
c. un polypeptide constitué d'une séquence d'acides aminés ayant au moins 95 % d'identité de séquence d'acides aminés avec la SEQ ID NO: 2;
d. un polypeptide comprenant une séquence d'acides aminés ayant au moins 95 % d'identité de séquence d'acides aminés avec la SEQ ID NO: 2.

3. Polypeptide Clorf54 pour une utilisation selon la revendication 1 ou 2, ladite activité de myokine comprenant une activité d'augmentation d'une fonction musculaire.
